# EUROPEAN PATENT APPLICATION

(11) **EP 4 726 038 A1**
(43) Date of publication of application: **15.04.2026**
(21) Application number: 24818762.7
(22) Date of filing: 07.06.2024
(51) Int. Cl.: C12N 15/11, C12N 15/113, A61K 9/127

(54) **EXPRESSION-ADJUSTABLE ENGINEERED RNA MOLECULE**

(30) Priority: 09.06.2023 WO PCT/CN2023/099512; 26.09.2023 WO PCT/CN2023/121779; 03.06.2024 CN 202410711025
(71) Applicant: Rinuagene Biotechnology Co., Ltd., Suzhou, Jiangsu 215000 (CN); Rinuagene International HK Limited, Kowloon Hong Kong 999077 (HK)
(72) Inventor: ZHANG, Weiguo, Suzhou, Jiangsu 215000 (CN); DONG, Yijie, Suzhou, Jiangsu 215000 (CN); CHEN, Rui, Suzhou, Jiangsu 215000 (CN); QI, Rui, Suzhou, Jiangsu 215000 (CN)
(74) Representative: Keller Schneider Patentanwaltsgesellschaft mbH
(86) International application number: PCT/CN2024/097944
(87) International publication number: WO 2024/251232

(57) **Abstract**

This application relates to an engineered RNA molecule, a DNA molecule encoding the engineered RNA molecule, and use of the engineered RNA molecule. The engineered RNA molecule comprises a Poly(A) tail sequence containing an miRNA binding site. The Poly(A) tail enables the accurate expression of a target gene in an organ, a tissue and/or a cell.

## Description

### CROSS-REFERENCE TO RELATED APPLICATIONS

This application claims priorities to Chinese Provisional Patent Application No. PCT/CN2023/099512 filed on June 9, 2023, Chinese Provisional Patent Application No. PCT/CN2023/121779 filed on September 26, 2023, and Chinese Provisional Patent Application No. 202410711025.6 filed on June 3, 2024. These prior applications are incorporated herein by reference in their entireties.

### TECHNICAL FIELD

This application relates to the technical field of biotechnologies, and specifically to an engineered RNA molecule comprising a polyadenylic acid (Poly(A)) tail. The Poly(A) tail enables the accurate expression of the engineered RNA molecule in a particular organ, tissue and/or cell.

### BACKGROUND

Deoxyribonucleic acid (DNA) and messenger ribonucleic acid (mRNA) are the main components in biological genetic materials, and mainly functions are to carry and transfer genetic information. In an eukaryotic cell, a complete mRNA mainly includes a 5' cap structure, a 5' untranslated region (5'UTR), a coding region (ORF), a 3' untranslated region (3'UTR), and 3' poly(A) tail structure. The 5' cap structure is mainly responsible for regulating the stability of the mRNA and the initiation of mRNA translation. The cap structure blocks the 5' end to prevent mRNA from being hydrolyzed by a nucleic acid exonuclease. Moreover, the cap structure can be recognized and bound by a cap binding protein (eIF-4E), to regulate the binding of mRNA with ribosome, thus initiating the translation process. The 5' UTR is a short sequence between the cap and the initiation codon, and a too short or too long 5' UTR is not conducive to the translation initiation of mRNA. It is generally believed that 3' UTR is mainly involved in post-transcriptional regulation, including regulating the in-vivo half-life of mRNA. For example, many microRNAs (miRNAs) can bind to 3' UTR of a target gene mRNA, and reduce the expression of the target gene by degradation or binding inhibition. 3'UTR is followed by a poly(A) sequence, which can prevent the degradation by an exonuclease. Additionally, the Poly(A) tail sequence can also bind to a poly(A) binding protein (PABP), and further recruit many proteins such as eIF4G, eIF4B, and Paip-1 to form a complex, which is involved in the regulation of mRNA stability and the initiation of translation. The Poly(A) tail of natural mRNA in eukaryotic cells is added by a Poly(A) polymerase after transcription. The polymerase has substrate specificity for adenylic acid during the evolution from fungi, plants to animals. Up to now, there is no clear evidence that a complex non-A motif with specific regulatory function can be formed in the natural Poly(A) tail structure.

The mRNA therapy has a wide scope of applications, and mainly includes the following directions according to different applications of the mRNA therapy: vaccines for infectious diseases, tumor immunotherapy, monoclonal antibody drugs and other protein drug substitutes, and gene editing. The success of mRNA vaccines during the COVID-19 pandemic also confirms the great value of the mRNA platform. The mRNA therapy usually delivers mRNA by lipid-based carrier systems (including liposomes and lipid nanoparticles (LNPs)). These lipid carriers generally encapsulate mRNA and improve the intracellular delivery and effectiveness of mRNA. LNP preparations represent a revolution in the art of nucleic acid delivery. LNP usually includes one or more cationic lipids and/or amino (ionizable) lipids, phospholipids, structural lipids (such as cholesterol) and/or lipids containing polyethylene glycol (PEGylated lipids). The cationic and/or ionizable lipids include, for example, lipids containing amines, which can be easily protonated.

Although the research on LNP-mediated nucleic acid delivery has made great progress, almost all of the current delivery systems have strong hepatotropism. Because the structure-effect relationship of the LNP delivery systems is not clear, it is difficult to rationally design delivery vectors that are highly specific for organs and tissues, especially for different cell types of the same organ and tissue, resulting in the widespread accumulation of mRNA-LNP in multiple organs, tissues and/or cell types. How to deliver mRNA to a specific organ, tissue and/or cell to realize the specific expression of the delivered mRNA in the specific organ, tissue and/or cell and reduce the side effects on other organs, tissues and/or cells is a main challenge for this kind of therapy.

miRNAs are small single-stranded, 19-25-nucleotide-long, non-coding RNA molecules found in plants, animals and some viruses, which are mainly responsible for mRNA degradation or silencing and post-transcriptional regulation of gene expression. They are complementary to and form a double-stranded sequence with a specific sequence in the 3'UTR of a target mRNA molecule through Watson-Crick base pairing, leading to the silencing of the mRNA molecule. The expression of miRNA is highly specific in organs, tissues, and cells. Examples of tissue-specific expression of miRNA include liver (miRNA-122) and spleen (miRNA-142), etc. The miRNA binding sequence is usually located in 3'UTR of mRNA. It has been reported in the art to integrate the miRNA binding sites into 3'UTR to improve the off-target expression.

In the field of mRNA, the first step to prepare mRNA drugs in vitro is to synthesize them by in-vitro transcription (IVT) using a linearized plasmid containing a designed product sequence as a template, where the Poly(A) tail is usually added downstream of 3'UTR by co-transcription. To add Poly(A) by co-transcription, a corresponding poly(dA:dT) sequence needed to be included in the template plasmid. However, the poly(dA:dT) repeat sequence in the plasmid is unstable in the process of replication in E.coli, and deletion mutation often occurs, which leads to the shortening of poly(dA:dT). This phenomenon is not conducive to a preparation process for large-scale production of a template plasmid for in-vitro transcription by fermentation. The Poly(A) truncation has a significant impact on the in-vivo stability and biological activity of mRNA. Therefore, the stability of plasmid replication of mRNA is an important factor in the investigation of mRNA drugs/vaccines.

In the art of mRNA, there is still an urgent need to develop quality- and activity-controllable mRNA vaccines or drugs that can be delivered to specific organs, tissues and/or cells and are convenient for industrial production, and tools and methods for regulating the expression of the delivered mRNA in specific organs, tissues and/or cells.

### SUMMARY

The polyA in natural has no miRNA binding site. In this application, the inventor creatively integrates a miRNA binding site into the Poly(A) tail, which not only enhances the specificity of mRNA expression in specific organs, tissues and/or cells, but also enhances the stability of plasmid replication of mRNA. Accordingly, the quality of the produced mRNA is controllable and the effect is stable.

Specifically, this application provides:
1. An engineered Poly(A) tail, comprising a miRNA binding site. The engineered Poly(A) tail may comprise one, two, or more miRNA binding sites.

In some embodiments, the sequences of the two or more miRNA binding sites are adjacent to each other or spaced by one or more bases.

2. The engineered Poly(A) tail according to Solution 1, comprising a structure of Formula (I) below:

nA-miRNA binding location-mA Formula (I);

where the miRNA binding location comprises one or more miRNA binding sites,
nA represents consecutive n adenylic acids (As) adjacent to the 5' end of the miRNA binding location,
mA represents consecutive m adenylic acids (As) adjacent to the 3' end of the miRNA binding location,
m and n are natural numbers, and m+n≤150, m+n≤120, m+n≤100, m+n≤80, m+n≤60, m+n≤30, m+n≤19, or m+n≤14. In some embodiments, the sum of m and n is: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, or 150.

In some embodiments, the one or more miRNA binding sites in the miRNA binding location are directly linked or linked by a spacer sequence. The spacer sequence consists of one or more nucleotides. In some embodiments, the spacer sequence consists of one or more non-A nucleotides.

In some embodiments, the miRNA binding location in Formula (I) consists of 1, 2, 3, 4, 5 or 6 miRNA binding sites. In some embodiments, the miRNA binding location in Formula (I) consists of one miRNA binding site. In some embodiments, the miRNA binding location in Formula (I) comprises two or more miRNA binding sites that are adjacent or linked by one or more nucleotides. In some embodiments, the miRNA binding location in Formula (I) comprises three or more miRNA binding sites that are adjacent or linked by one or more nucleotides. In some embodiments, the Poly(A) tail has only one structure of Formula (I). In some embodiments, the Poly(A) tail is the structure of Formula (I).

3. The engineered Poly(A) tail according to Solution 2, where n=0 or n≥1.

4. The engineered Poly(A) tail according to Solution 2, where m=0.

5. The engineered Poly(A) tail according to Solution 2, where n≤80, n≤60, n≤30, n≤19, n≤14, or n≤10. In some embodiments, 14≤n≤30, 14≤n≤19, or 19≤n≤30. In some embodiments, n is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 or 80.

6. The engineered Poly(A) tail according to any one of Solutions 1 to 5, where the Poly(A) tail has a length of 80 to 240 nt, for example, 100 to 200 nt, 101 to 150 nt, 120 to 150 nt, 130 to 140 nt, 123 to 135 nt, or 125 139 nt. In some embodiments, the Poly(A) tail has a length of 81 nt, 82 nt, 83 nt, 84 nt, 85 nt, 86 nt, 87 nt, 88 nt, 89 nt, 90 nt, 91 nt, 92 nt, 93 nt, 94 nt, 95 nt, 96 nt, 97 nt, 98 nt, 99 nt, 100 nt, 101 nt, 102 nt, 103 nt, 104 nt, 105 nt, 106 nt, 107 nt, 108 nt, 109 nt, 110 nt, 111 nt, 112 nt, 113 nt, 114 nt, 115 nt, 116 nt, 117 nt, 118 nt, 119 nt, 120 nt, 121 nt, 122 nt, 123 nt, 124 nt, 125 nt, 126 nt, 127 nt, 128 nt, 129 nt, 130 nt, 131 nt, 132 nt, 133 nt, 134 nt, 135 nt, 136 nt, 137 nt, 138 nt, 139 nt, 140 nt, 141 nt, 142 nt, 143 nt, 144 nt, 145 nt, 146 nt, 147 nt, 148 nt, 149 nt, 150 nt, 151 nt, 152 nt, 153 nt, 154 nt, 155 nt, 156 nt, 157 nt, 158 nt, 159 nt, 160 nt, 161 nt, 162 nt, 163 nt, 164 nt, 165 nt, 166 nt, 167 nt, 168 nt, 169 nt, 170 nt, 171 nt, 172 nt, 173 nt, 174 nt, 175 nt, 176 nt, 177 nt, 178 nt, 179 nt, 180 nt, 181 nt, 182 nt, 183 nt, 184 nt, 185 nt, 186 nt, 187 nt, 188 nt, 189 nt, 190 nt, 191 nt, 192 nt, 193 nt, 194 nt, 195 nt, 196 nt, 197 nt, 198 nt, 199 nt, 200 nt, 201 nt, 202 nt, 203 nt, 204 nt, 205 nt, 206 nt, 207 nt, 208 nt, 209 nt, 210 nt, 211 nt, 212 nt, 213 nt, 214 nt, 215 nt, 216 nt, 217 nt, 218 nt, 219 nt, 220 nt, 221 nt, 222 nt, 223 nt, 224 nt, 225 nt, 226 nt, 227 nt, 228 nt, 229 nt, 230 nt, 231 nt, 232 nt, 233 nt, 234 nt, 235 nt, 236 nt, 237 nt, 238 nt, or 239 nt.

7. The engineered Poly(A) tail according to any one of Solutions 1 to 6,
further comprising, at the 3' side of the structure of Formula (I), a tail fragment directly linked to the 3' end of the structure of Formula (I), where preferably the 5'-terminal nucleotide of the tail fragment is not A; and/or
further comprising, at the 5' side of the structure of Formula (I), a head fragment directly linked to the 5' end of the structure of Formula (I), where preferably the 3'-terminal nucleotide of the head fragment is not A.

In some embodiments, the tail fragment or the head fragment consists of one or more non-A nucleotides and multiple As, for example, including 1, 2, 3, 4, 5, 6, 7, 8, 9, 10, 11, 12, 13, 14, 15, 16, 17, 18, 19, or 20 non-A nucleotides.

In some embodiments, the tail fragment or the head fragment includes p nucleotides, where p≤80, p≤60, p≤30, p≤19, or p≤14. In some embodiments, p is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, or 79.

In some embodiments, the tail fragment or the head fragment consists of one or more elements c and/or one or more elements d and one or more A.

Element c is a non-A nucleotide, for example, T, U, C or G.

Element d consists of any two or more consecutive nucleotides, and the 5'- and 3'-terminal nucleotides of element d are non-A nucleotides.

element d has a length in the range of 2 nt≤d≤30 nt, preferably 6 nt≤d≤20 nt, and further preferably 6 nt≤d≤12 nt.

Element c and element d are not contiguous.

In some embodiments, element d has a length of 2 nt, 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt or 30 nt.

In some embodiments, element d consists of any two or more consecutive nucleotides, and element d does not include more than three consecutive A, where the nucleotide is selected from A, U, C, and G nucleotide. The 5'- and 3'-terminal nucleotides of element d are non-A nucleotides. Preferably, element d has a length in the range of 2 nt≤d≤30 nt, preferably 6 nt≤d≤20 nt, and further preferably 6 nt≤d≤12 nt.

In some embodiments, the number of element c is 0, 1, 2 to 10, 3 to 8, 4 to 6, or 2 to 5, for example, 7. In some embodiments, the number of element c is 0, 1, 2, 3, 4, 5, 6, 7, 8, 9, or 10.

In some embodiments, the number of element d is 0 to 5, and preferably 1 to 3, for example, 2 or 4. In some embodiments, the number of element d is 0, 1, 2, 3, 4, or 5.

In some embodiments, when both element c and element d are present, the total number of element c and element d is 2 to 15, preferably 3 to 5, and further preferably 3. In some embodiments, the total number of element c and element d is 4, 5, 6, 7, 8, 9, 10, 11, 12, 13 or 14.

In some embodiments, the Poly(A) tail includes, at 1/2 close to the 3' end, one or more non-A nucleotides.

In some embodiments, the Poly(A) tail includes, at 1/3 close to the 3'end, one or more non-A nucleotides.

In some embodiments, the Poly(A) tail includes, at 1/4 close to the 3' end, one or more non-A nucleotides.

The engineered Poly(A) tail according to any one of the preceding solutions, comprising, at the 5' side of the miRNA binding location, 0 to 60, 0 to 30, 0 to 20, 0 to 14, or 0 to 10 nucleotides, for example, 1, 2, 3, 4, 5, 6, 7, 8, 9, 10, 11, 12 or 13 nucleotides.

8. The engineered Poly(A) tail according to any one of Solutions 1 to 7, formed by inserting the miRNA binding location into a Poly(A) tail structure shown below:
element a-element c-element b-element c-element b-element c-element b-element c-element b;
element b-element c-element b-element c-element a-element d-element b-element c-element b-element c-element b;
element b-element c-element b-element c-element b-element d-element a-element c;
element a-element d-element b-element c-element b-element c-element b; or
element b-element c-element b-element c-element b-element d-element a;
element a-element d-element b;
element a-element d-element a;
element b-element d-element a;
element b-element d-element b;
element a-element d-A
element a-element c-A
where optionally:
   1) the miRNA binding location replaces any 1, 2, 3, 4, 5 or more elements;
   2) the miRNA binding location is inserted to any two elements; or
   3) the miRNA binding location is inserted into any two A in element a or element b.

In the Poly(A) tail structure, element a consists of multiple consecutive adenylic acid (A) nucleotides, and element a has a length in the range of 100 nt≥a>30 nt and preferably 80 nt≥a≥60 nt;
element b consists of multiple consecutive A nucleotides, and element b has a length in the range of 10 nt≤b≤30 nt and preferably 14 nt≤b≤20 nt;
element c consists of a non-A nucleotide;
element d consists of any two or more consecutive nucleotides, and element d does not include more than three consecutive A, where the nucleotide is selected from A, T, C, or G nucleotide. The 5'- and 3'-terminal nucleotides of element d are non-A nucleotides. Preferably, element d has a length in the range of 2 nt≤d≤30 nt, preferably 6 nt≤d≤20 nt, and further preferably 6 nt≤d≤12 nt.

In some embodiments, element a is 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, 50 nt, 51 nt, 52 nt, 53 nt, 54 nt, 55 nt, 56 nt, 57 nt, 58 nt, 59 nt, 60 nt, 61 nt, 62 nt, 63 nt, 64 nt, 65 nt, 66 nt, 67 nt, 68 nt, 69 nt, 70 nt, 71 nt, 72 nt, 73 nt, 74 nt, 75 nt, 76 nt, 77 nt, 78 nt, 79 nt, 80 nt, 81 nt, 82 nt, 83 nt, 84 nt, 85 nt, 86 nt, 87 nt, 88 nt, 89 nt, 90 nt, 91 nt, 92 nt, 93 nt, 94 nt, 95 nt, 96 nt, 97 nt, 98 nt, 99 nt, or 100 nt.

In some embodiments, element b is 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt or 30 nt.

In some embodiments, element d is 2 nt, 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt or 30 nt.

9. The engineered Poly(A) tail according to any one of Solutions 1 to 7, formed by inserting the miRNA binding location into a Poly(A) tail structure described in WO2020074642A1, WO2022028559A1 and US_10717982.

10. The engineered Poly(A) tail according to any one of Solutions 1 to 9, where element c is G.

11. The engineered Poly(A) tail according to any one of Solutions 1 to 10, where element d comprises a palindrome sequence.

12. The engineered Poly(A) tail according to any one of Solutions 1 to 11, where element d has a length of 6 nt.

13. The engineered Poly(A) tail according to any one of Solutions 1 to 12, where element d is one or more selected from: GATATC (SEQ ID NO: 60), GTATAC (SEQ ID NO: 61), GAATCT (SEQ ID NO: 62), GCATATGACT (SEQ ID NO: 63), and GATATCGTATAC (SEQ ID NO: 64).

14. The engineered Poly(A) tail according to any one of Solutions 1 to 12, where element d is as shown in GATATC (SEQ ID NO: 60).

15. The engineered Poly(A) tail according to any one of Solutions 1 to 14, where the ratio of nucleotide number included in the Poly(A) tail at the 5' side of the miRNA binding location to nucleotide number included in the Poly(A) tail at the 3' side of the miRNA binding location is less than 1/1, 1/2, 1/3, 1/4, 1/5, 1/6, 1/7, 1/8, 1/9, or 1/10.

16. The engineered Poly(A) tail according to any one of Solutions 6 to 15, where the tail fragment has a structure of: element d-element b-element c-element b-element c-element b or element c-element b-element c-element b, preferably the tail fragment has a structure of: element d-19A-element c-19A-element c-17A or element c-19A-element c-17A, and further preferably the tail fragment has a structure of: GATATC-19A-G-19A-G-17A. In some embodiments, the engineered Poly(A) tail comprises no head fragment. In some embodiments, the engineered Poly(A) tail comprises the tail fragment and the structure of Formula (I). In some embodiments, the engineered Poly(A) tail comprises the head fragment, the tail fragment, and the structure of Formula (I).

The engineered Poly(A) tail according to any one of Solutions 1 to 15, having any structure selected from:
1) nA-miRNA binding location -mA, where 150≥m+n≥50, and preferably, m+n=120 or m+n=60;
2) nA-miRNA binding location-mA-element d-element b-element c-element b-element c-element b, where 80≥m+n≥60, and preferably, m+n=60;
3) element a-element d-nA-miRNA binding location-mA-element c-element b-element c-element b, where
   30≥m+n≥10, and preferably m+n=19;
4) nA-miRNA binding location-mA-element c-element b-element c-element b, where 110≥m+n≥70, and preferably, m+n=79;
5) nA-miRNA binding location-mA-element c-element b-element c-element b-element d-element a or element b-element c-nA-miRNA binding location -mA-element c-element b-element d-element a, where 19≥m+n≥14, and preferably, m+n=19.

The engineered Poly(A) tail according to any one of Solutions 1 to 15, having a structure of:
nA-miRNA binding location-mA-element d-element b-element c-element b-element c-element b, where 80≥m+n≥60, and preferably, m+n=60; further preferably, 30≥n≥14; and further preferably 19≥n≥14.

The engineered Poly(A) tail according to any one of Solutions 1 to 15, having any structure selected from:
1) nA-miRNA binding location-mA, where m+n=120, or m+n=100, or m+n=60;
2) nA-miRNA binding location-mA-element d-19A-G-19A-G-17A, where m+n=60;
3) 60A-element d-nA-miRNA binding location-mA-G-19A-G-17A, where m+n=19;
4) nA-miRNA binding location-mA-G-19A-G-17A, where m+n=79; and
5) nA-miRNA binding location-mA-G-19A-G-19A-element d-60A or 19A-G-nA-miRNA binding location-mA-G-19A-element d-60A, where m+n=19.

The engineered Poly(A) tail according to any one of Solutions 1 to 15, having a structure of:
nA-miRNA binding location-mA-element d-19A-G-19A-G-17A, where m+n=60 and 30≥n≥14; and preferably, 19≥n≥14.

The engineered Poly(A) tail according to any one of Solutions 1 to 15, having any structure selected from:
1) nA-miRNA binding location-mA, where m+n=120 or m+n=100;
2) nA-miRNA binding location-mA-SEQ ID NO: 60-19A-G-19A-G-17A, where where m+n=60;
3) 60A-SEQ ID NO: 60-nA-miRNA binding location-mA-G-19A-G-17A, where m+n=19;
4) nA-miRNA binding location-mA-G-19A-G-17A, where m+n=79; and
5) nA-miRNA binding location-mA -G-19A-G-19A- SEQ ID NO: 60-60A or 19A-G- nA-miRNA binding location-mA-G-19A-element d-60A, where m+n=19.

The engineered Poly(A) tail according to any one of Solutions 1 to 15, having a structure of:
nA-miRNA binding location-mA-SEQ ID NO: 60-19A-G-19A-G-17A, where m+n=60 and 30≥n≥14, and preferably, 19≥n≥14.

17. The engineered Poly(A) tail according to any one of Solutions 1 to 16, where the miRNA binding to the miRNA binding location is one or more selected from:
miR-142, miR-122, miR-126, miR-148a, miR-133, miR-206, miR-208, miR-17-92, miR-16, miR-21, miR-223, miR-24, miR-27, let-7, miR-30c, miR-1d, miR-149, miR-192, miR-194 and miR-204.

In some embodiments, the miRNA is one or more selected from miR-142, miR-122, miR-126, and miR-148a.

In some embodiments, the miRNA is one or more selected from miR-142-3p, miR-122-5p, miR-126-3p, and miR-148a-3p.

In some embodiments, the miRNA includes or consists of: miR142 and miR-122, miR142 and miR-126, miR-142 and miR-148a, miR-122 and miR-126, miR-122 and miR-148a, or miR-126 and miR-148a.

In some embodiments, the miRNA includes or consists of: miR-142, miR-122 and miR-126, miR-142, miR-122 and miR-148a, miR-142, miR-126 and miR-148a, or miR-122, miR-126 and miR-148a.

In some embodiments, the miRNA includes or consists of: miR-142, miR-122, miR-126 and miR-148a.

In some embodiments, the miRNA includes miR142 and miR-122 or consists of miR142 and miR-122; and in the miRNA binding location of the Poly(A), the miR142 binding site is located at the 5' side of the miR-122 binding site or the miR142 binding site is located at the 3' side of the miR-122 binding site, and the miR142 binding site and the miR-122 binding site are adjacent to each other.

In some embodiments, the miRNA includes miR142 and miR-122; and in the binding location of the Poly(A), the miR142 binding site is located at the 5' side of the miR-122 binding site or the miR142 site is located at the 3' side of the miR-122 site, and the miR142 binding site and the miR-122 binding site are spaced apart by one or more nucleotides.

In some embodiments, the miRNA includes miR142 and miR-126 or consists of miR142 and miR-126; and in the binding location of the Poly(A), the miR142 binding site is located at the 5' side of the miR-126 binding site or the miR142 site is located at the 3' side of the miR-126 site, and the miR142 binding site and the miR-126 binding site are adjacent to each other.

In some embodiments, the miRNA includes miR142 and miR-126; and in the binding location of the Poly(A), the miR142 binding site is located at the 5' side of the miR-126 binding site or the miR142 site is located at the 3' side of the miR-126 site, and the miR142 binding site and the miR-126 binding site are spaced apart by one or more nucleotides.

In some embodiments, the miRNA includes miR-142 and miR-148a or consists of miR-142 and miR-148a; and in the binding location of the Poly(A), the miR-142 binding site is located at the 5' side of the miR-148a binding site or the miR-142 binding site is located at the 3' side of the miR-148a binding site, and the miR-142 binding site and the miR-148a binding site are adjacent to each other.

In some embodiments, the miRNA includes miR-142 and miR-148a; and in the binding location of the Poly(A), the miR-142 binding site is located at the 5' side of the miR-148a binding site or the miR-142 binding site is located at the 3' side of the miR-148a binding site, and the miR-142 binding site and the miR-148a binding site are spaced apart by one or more nucleotides.

In some embodiments, the miRNA includes miR-122 and miR-126 or consists of miR-122 and miR-126; and in the binding location of the Poly(A), the miR-122 binding site is located at the 5' side of the miR-126 binding site or the miR-122 binding site is located at the 3' side of the miR-126 binding site, and the miR-122 binding site and the miR-126 binding site are adjacent to each other.

In some embodiments, the miRNA includes miR-122 and miR-126; and in the binding location of the Poly(A), the miR-122 binding site is located at the 5' side of the miR-126 binding site or the miR-122 binding site is located at the 3' side of the miR-126 binding site, and the miR-122 binding site and the miR-126 binding site are spaced apart by one or more nucleotides.

In some embodiments, the miRNA includes miR-122 and miR-148a or consists of miR-122 and miR-148a; and in the binding location of the Poly(A), the miR-122 binding site is located at the 5' side of the miR-148a binding site or the miR-122 binding site is located at the 3' side of the miR-148a binding site, and the miR-122 binding site and the miR-148a binding site are adjacent to each other.

In some embodiments, the miRNA includes miR-122 and miR-148a; and in the binding location of the Poly(A), the miR-122 binding site is located at the 5' side of the miR-148a binding site or the miR-122 binding site is located at the 3' side of the miR-148a binding site, and the miR-122 binding site and the miR-148a binding site are spaced apart by one or more nucleotides.

In some embodiments, the miRNA includes miR-126 and miR-148a or consists of miR-126 and miR-148a; and in the binding location of the Poly(A), the miR-126 binding site is located at the 5' side of the miR-148a binding site or the miR-126 binding site is located at the 3' side of the miR-148a binding site, and the miR-126 binding site and the miR-148a binding site are adjacent to each other.

In some embodiments, the miRNA includes miR-126 and miR-148a; and in the binding location of the Poly(A), the miR-126 binding site is located at the 5' side of the miR-148a binding site or the miR-126 binding site is located at the 3' side of the miR-148a binding site, and the miR-126 binding site and the miR-148a binding site are spaced apart by one or more nucleotides.

In some embodiments, the miRNA includes miRNA-142, miR-148a and miR-126; and in the miRNA binding location of the Poly(A), the miRNA binding sites are arranged, from 5' to 3', in a sequence of:
miR-126 binding site, miR-148a binding site and miR-142 binding site;
miR-148a binding site, miR-142 binding site and miR-126 binding site;
miR-142 binding site, miR-148a binding site and miR-126 binding site;
miR-126 binding site, miR-142 binding site and miR-148a binding site;
miR-148a binding site, miR-126 binding site and miR-142 binding site; or
miR-142 binding site, miR-126 binding site and miR-148a binding site;
where the miR-126 binding site, the miR-148a binding site, and the miR-142 binding site are adjacent to each other.

In some embodiments, the miRNA includes miRNA-142, miR-148a and miR-126; and in the miRNA binding location of the Poly(A), the miRNA binding sites are arranged, from 5' to 3', in a sequence of:
miR-126 binding site, miR-148a binding site and miR-142 binding site;
miR-148a binding site, miR-142 binding site and miR-126 binding site;
miR-142 binding site, miR-148a binding site and miR-126 binding site;
miR-126 binding site, miR-142 binding site and miR-148a binding site;
miR-148a binding site, miR-126 binding site and miR-142 binding site; or
miR-142 binding site, miR-126 binding site and miR-148a binding site;
where the miR-126 binding site, the miR-148a binding site, and the miR-142 binding site are spaced apart from each other by one or more nucleotides.

In some embodiments, the miRNA includes miRNA-142, miR-148a and miR-126; and in the miRNA binding location of the Poly(A), the miRNA binding sites are arranged, from 5' to 3', in a sequence of:
miR-126 binding site, miR-148a binding site and miR-142 binding site;
miR-148a binding site, miR-142 binding site and miR-126 binding site;
miR-142 binding site, miR-148a binding site and miR-126 binding site;
miR-126 binding site, miR-142 binding site and miR-148a binding site;
miR-148a binding site, miR-126 binding site and miR-142 binding site; or
miR-142 binding site, miR-126 binding site and miR-148a binding site;
where two contiguous miRNA binding sites of the miR-126 binding site, the miR-148a binding site and the miR-142 binding site are adjacent to each other, and the other two miRNA binding sites are spaced apart by one or more nucleotides.

The engineered Poly(A) tail according to any one of preceding solutions, having any structure selected from:
1) nA-miRNA binding location-mA, where n=0, m=120, or m=100, or m=60;
2) nA-miRNA binding location-mA-element d-19A-G-19A-G-17A, where m+n=60, n≤20, or n≤14, or n≤10;
3) nA-miRNA binding location-mA-element d-19A-G-19A-G-17A, where n=0, and m=60;
4) nA-miRNA binding location-mA-element d-19A-G-19A-G-17A, where m+n=60, 14≤n≤30; and preferably 14≤n≤19;
5) nA-miRNA binding location-mA-G-19A-G-17A, where n=60, and m=19;
where preferably element d is a palindrome sequence, and further preferably the element d has a polynucleotide sequence as shown in any one of SEQ ID NOs: 60 to 64.

The engineered Poly(A) tail according to any one of preceding solutions, having any structure selected from:
1) nA-miRNA binding location-mA, where n=0, and m=120, or m=100, or m=60, and the miRNA binding location is the miR-122, miR-126 or miR-142 binding site;
2) nA-miRNA binding location-mA-element d-19A-G-19A-G-17A, where n=14 and m=46, and the miRNA binding location is the miR-122, miR-126 or miR-142 binding site;
3) nA-miRNA binding location-mA-element d-19A-G-19A-G-17A, where n=19 and m=41, and the miRNA binding location is the miR-122, miR-126 or miR-142 binding site;
4) nA-miRNA binding location-mA-element d-19A-G-19A-G-17A, where n=30 and m=30, and the miRNA binding location is the miR-122, miR-126 or miR-142 binding site;
5) nA-miRNA binding location-mA-G-19A-G-17A, where n=60 and m=19, and the miRNA binding location is the miR-122, miR-126 or miR142 binding site; and
6) nA-miRNA binding location-mA-element d-19A-G-19A-G-17A, where n=0 and m=60, the miRNA binding location is the miR-142, miR-122, miR-126, miR-148a, miR-142-miR122, miR142-miR126-miR148a, miR142-miR148a-miR126; miR126-miR142-miR148a; miR126-miR148a-miR142, miR148a-miR142-miR126, or miR148a-miR126-miR142 binding site;
7) nA-miR-122 binding location-mA-element d-19A-G-19A-G-17A, where n=1, 2, 3, 4, 5, 6, 7, 8, 9 or 10, and n+m=60;
8) nA-miR-142 binding location -mA-element d-19A-G-19A-G-17A, where n=1, 2, 3, 4, 5, 6, 7, 8, 9 or 10, and n+m=60,
9) nA-miR-126 binding location -mA-element d-19A-G-19A-G-17A, where n=1, 2, 3, 4, 5, 6, 7, 8, 9 or 10, and n+m=60; or
10) nA-miR-148a binding location-mA-element d-19A-G-19A-G-17A, where n=1, 2, 3, 4, 5, 6, 7, 8, 9 or 10, and n+m=60,
where preferably element d is a palindrome sequence, and further preferably element d has a polynucleotide sequence as shown in any one of SEQ ID NOs: 60 to 64 and preferably as shown in SEQ ID NO: 60.

In some embodiments, the miRNA binding location of the engineered Poly(A) tail comprises one, two, three, four or more miRNA binding site sequences selected from: SEQ ID NO: 4, SEQ ID NO: 17, SEQ ID NO: 32, or SEQ ID NO: 53. In some embodiments, the miRNA binding location of the engineered Poly(A) tail comprises one, two, three, four or more miRNA binding site sequences selected from: SEQ ID NO: 4, SEQ ID NO: 17, SEQ ID NO: 32, or SEQ ID NO: 53 and 0, 1 or more nucleotides between the miRNA binding sites.

18. The engineered Poly(A) tail according to any one of Solutions 1 to 17, where the sequence of the Poly(A) tail comprises or is any one selected from the following polynucleotide sequences: SEQ ID NOs: 6-16, SEQ ID NOs: 19-31, SEQ ID NOs: 33-52, SEQ ID NOs: 54-59, and SEQ ID NOs: 65-71.

19. An engineered RNA molecule, which is an mRNA or a non-coding RNA, and comprising the Poly(A) tail according to any one of Solutions 1 to 18 and a target gene sequence located at the 5' side of the Poly(A) tail. In some embodiments, nA in the structure of Formula (I) in the Poly(A) tail is directly linked to 3'UTR.

20. An engineered DNA molecule, encoding the engineered Poly(A) tail according to any one of Solutions 1 to 18, or the engineered RNA sequence according to Solution 19.

21. The engineered DNA molecule according to Solution 20, which is a plasmid or viral vector.

22. A DNA-RNA hybrid molecule, carrying the same genetic information as the engineered Poly (A) tail according to any one of Solutions 1 to 18, the engineered RNA molecule according to Solution 19, or the engineered DNA molecule according to Solution 20 or 21.

23. An engineered cell, comprising the engineered Poly(A) tail according to any one of Solutions 1 to 18, the engineered RNA molecule according to Solution 19, the engineered DNA molecule according to Solution 20 or 21, or the hybrid molecule according to Solution 22, where the cell is an eukaryotic or a prokaryotic cell.

24. The engineered cell according to Solution 23, where the prokaryotic cell is *E. coli.*

25. Use of the engineered Poly(A) tail according to any one of Solutions 1 to 18 in enabling the low expression of a target gene in a particular organ, tissue and/or cell, where the particular organ, tissue and/or cell highly expresses the miRNA; or
use of the engineered Poly(A) tail according to any one of Solutions 1 to 18 in enabling the specific expression of a target gene in a particular organ, tissue and/or cell, where preferably, the organ or tissue is preferably a liver or spleen organ or tissue; and further preferably the cell is hepatic parenchymal cells.

26. Use of the engineered DNA molecule according to Solution 20 or 21 or the hybrid molecule according to Solution 22 in enabling the more conservative replication of a DNA coding sequence of the Poly(A) tail in a host cell.

27. The use according to Solution 26, wherein the host cell is a prokaryotic cell, and preferably *E. coli.*

28. A lipid nanoparticles, comprising the engineered Poly(A) tail according to any one of Solutions 1 to 18, the engineered RNA molecule according to Solution 19, the engineered DNA molecule according to Solution 20 or 21, or the hybrid molecule according to Solution 22.

29. A virion, comprising the engineered Poly(A) tail according to any one of Solutions 1 to 18, the engineered RNA molecule according to Solution 19, the engineered DNA molecule according to Solution 20 or 21, or the hybrid molecule according to Solution 22.

It is known to those skilled in the art that at the 5' side of the 5' terminal nucleotide of the miRNA binding location, 0, 1 or more non-A nucleotides may be further included. This technical solution is also contemplated in the scope of this application, or be regarded as an equivalent solution of this application.

It is to be understood that aspects and embodiments of this application described herein include those that "comprise", "consist of" and "consist essentially of......". Preferred embodiments of this application have been described in detail above. However, this application is not limited thereto. Various simple modifications can be made to the technical solution of this application within the technical concept of this application, including the combinations of various technical features in any other suitable ways. These simple modifications and combinations should also be regarded as disclosure in this application and are all embraced in the scope of protection of this application.

### BRIEF DESCRIPTION OF THE DRAWINGS

Figs. 1A-1B show the composition of 5 Poly(A) containing a miR-142/miR-122 binding site independently designed in the present invention.
Figs. 2A-2B show the absolute numerical results of fluorescence imaging of dissected mouse spleen/liver after the luciferase mRNA with 5 Poly(A) containing a miR-142 binding site independently designed in the present invention is expressed in mice.
Figs. 3A-3B show the absolute numerical results of fluorescence imaging of dissected mouse spleen/liver after the luciferase mRNA with 5 Poly(A) containing a miR-122 binding site independently designed in the present invention is expressed in mice.
Fig. 4 shows the statistical result of missed bases of a luciferase DNA plasmid with various poly(A) containing the miRNA binding site miR-142/miR-122 independently designed in the present invention in E. coli DH5α.
Fig. 5 shows the absolute numerical results of enzyme activity detection for luciferase mRNA with 8 Poly(A) containing a miR-142 binding site independently designed in the present invention in the mammalian cell line Raw 264.7.
Fig. 6 shows the absolute numerical results of enzyme activity detection for luciferase mRNA with poly(A) containing a miR-142 binding site and a miR-122 binding site in the mammalian cell line Raw 264.7.
Figs. 7A-7D show the absolute numerical results of enzyme activity detection for luciferase mRNA with a poly(A) variant independently designed in the present invention in the mammalian cell line Raw 264.7/LSEC/HSC/AML.
Figs. 8A-8C show the reduction ratio of the expression of luciferase mRNA with a poly(A) variant independently designed in the present invention in Raw 264.7/LSEC/HSC to the expression in hepatic parenchymal cells AML.

### DETAILED DESCRIPTION

This application provides a tool to realize the specific expression in a particular organ, tissue and/or cell. That is, by integrating a miRNA binding site into a Poly(A) tail, the mRNA expression in an organ, tissue and/or cell where the mRNA expression is undesired is specifically turned off, to reduce the off-target toxic side effect due to the systematic expression of mRNA.

This application further provides a method for stably amplifying a template DNA for transcription of Poly(A) tail in vitro, to reduce, when the DNA is replicated in a large amount in the cells, the frequency of mutation in the template sequence for transcription of Poly(A) tail. In this way, a large amount of RNA containing a Poly(A) tail with a definite sequence is obtained based on the DNA. On this basis, RNA, such as mRNA, that is engineered to have a Poly(A) tail of a specific function, can be fermented in vitro to realize large-scale production.

### Terms

In this application, "element c", "element d", nA, mA, tail fragment, head fragment, miRNA binding site and miRNA binding location in the Poly(A) tail all can be indicated by the term "element ". The "miRNA binding location", "nA", "mA", "element c" and "element d" do not overlap with or contain each other. The "miRNA binding location", "nA", "mA", "element c" and "element d" can be determined as follows.

A miRNA binding site(s) in the Poly(A) tail is/are determined by sequence alignment. N consecutive As are determined as nA by determining an A, most contiguous to the 5' side of a miRNA binding site in all the miRNA binding site(s) that is closest to the 5' end of the Poly(A) tail, as the 3' end of the nA. M consecutive As are determined as mA by determining an A, most contiguous to the 3' side of a miRNA binding site in all the miRNA binding site(s) that is closest to the 3' end of the Poly(A) tail, as the 5' end of the mA. The moiety between nA and mA is the miRNA binding location. If the Poly(A) tail has a polynucleotide or a polynucleotide sequence at the 5' side of nA, the polynucleotide or polynucleotide sequence is a head fragment; and if the Poly(A) tail has a polynucleotide or a polynucleotide sequence at the 3' side of mA, the polynucleotide or polynucleotide sequence is a tail fragment. "Element d" and "element c" are determined as follows. Element d is determined from a target sequence. Element d is a fragment comprising a non-A base in the target sequence and consists of any two or more consecutive nucleotides. The 5' and 3'-terminal nucleotides of element d are non-A nucleotides, element d does not include more than three consecutive As, and both the 5' and 3' ends of element d are adjacent to at least 2 As. After element d is determined, all non-A bases are determined to be "element C" from the parts other than element D in the target sequence. In some embodiments, the Poly(A) tail comprises multiple miRNA binding locations, and the miRNA binding sites in each binding location are directly linked or linked by a non-A base. In some embodiments, the Poly(A) tail comprises multiple miRNA binding locations, and the miRNA binding sites in each binding location are directly linked or linked by a nucleic acid, where the nucleic acid sequence does not include more than three consecutive As.

As used herein, "miRNA" or microRNA, is a short non-coding RNA having a length of generally less than 22 bp. It binds to a target sequence (that is, miRNA binding site) on mRNA, to exert a post-transcriptional regulatory effect. As used herein, preferably, the "miRNA binding site" is a miRNA binding site that down-regulates mRNA expression after binding to miRNA.

Herein, "coding" means that i) a DNA sequence contains genetic information that can be transcribed into a RNA molecule, and/or ii) a RNA molecule contains genetic information that can be translated into an amino acid sequence. Therefore, as used herein, a "coding sequence" can be used to refer to a ribonucleic acid (RNA) sequence or a fragment thereof in an mRNA precursor or mature mRNA that can be translated into a protein, or a complementary sequence or a fragment of a deoxyribonucleic acid (DNA) sequence used as a template to transcribe into the mRNA precursor or mature mRNA. Additionally, the "coding sequence" in this application may further include a polynucleotide sequence encoding a protein, a functional nucleic acid, or a fragment thereof, for example, miRNA, shRNA, dsRNA, guide RNA, Poly(A) tail, 5'UTR, or 3'UTR. A DNA molecule containing genetic information that can be transcribed into a RNA molecule is called a "coding nucleic acid" of the RNA molecule. A RNA molecule containing genetic information that can be translated into an amino acid sequence is called a "coding nucleic acid" of the amino acid sequence.

In this application, all the nucleotides in a polynucleotide sequence are numbered from the 5' end to the 3' end, that is, the 5'-terminal nucleotide is the first nucleotide and the 3'-terminal nucleotide is the last nucleotide. Unless otherwise specified, the "5' terminus" and "5' end" are interchangeable, and the "3' terminus" and "3' end" are interchangeable. Particularly, the "5' end" and "3' end" can be used to describe the position of the first and last nucleotide of a nucleic acid sequence or a segment of a nucleic acid sequence, respectively. Additionally, "5'" and "3'" are especially used to describe the relative position relationship, between nucleotides, between nucleotide segments, or between a nucleotide and a nucleotide segment in the same nucleic acid sequence. For example, the "5' side" is used to describe the relative position relationship between two sequences in the same polynucleotide sequence that do not overlap with each other. When one sequence is described to be located at the 5' side of other sequence, it means that the one sequence is closer to the "5' end" of a polynucleotide sequence than the other sequence. Similarly, when one sequence is described to be located at the 3' side of other sequence, it means that the one sequence is closer to the "3' end" of a polynucleotide sequence than the other sequence, and the one sequence and the other sequence do not contain overlapping parts with each other. Additionally, as used herein, the "5' part" refers to a half of a polynucleotide sequence close to the 5' end, divided by the "central position" of the polynucleotide sequence. The "3' part" refers to a half of a polynucleotide sequence close to the 3' end, divided by the central position of the polynucleotide sequence. In this application, the number of nucleotides from the "central position" to the 5' end and to the 3' end is equal.

As used herein, "adjacent" means that there is no nucleotide or base insertion between two elements of a nucleic acid molecule (such as a Poly(A) tail) (unless otherwise specified, the term "nucleotide" and the term "base" are used interchangeably in this application). That is, the first nucleotide of the 3' side at the 3' end of the polynucleotide sequence of one of the two elements is the 5'-terminal nucleotide of the other element. For example, "consecutive n As adjacent to the 5' end of the miRNA binding site" means that the consecutive n As are located at the 5' side of the miRNA binding site, and no other nucleotides or bases are inserted between the 5'-terminal nucleotide of the miRNA binding site and the n As. For example, "adjacent to the 3' end of the miRNA binding site" means that the consecutive n As are located at the 3' side of the miRNA binding site, and no other nucleotides or bases are inserted between the 3'-terminal nucleotide of the miRNA binding site and the n As. Additionally, "adjacent" can also be used to describe the position relationship between multiple elements, which means that each element of the multiple elements is adjacent to any other element of the multiple elements, and no other elements or nucleotides other than the multiple elements are included between any two elements of the multiple elements.

In this application, when the position relationship between two or more elements is described as "non-contiguous", it means that two or more elements are not adjacent to each other. In other words, at least one or more other nucleotides or bases other than those of the two elements are included between the two or more elements.

As used herein, when the term "conservative" is used to describe the replication of a nucleic acid molecule, it means that the probability of mutation during the replication process is low. In this context, "conservative" is a relative concept. For example, in the description that "the DNA coding sequence of the Poly(A) tail is used to make the replication of the DNA molecule encoding the RNA more conservative in the host cell", it means that after a parent DNA molecule encoding the RNA is replicated into a daughter DNA molecule, if the RNA molecule contains the Poly(A) tail, the probability that the daughter DNA molecule has 100% sequence identity with the parent DNA molecule is higher, compared with the coding DNA of the RNA molecule that does not contain the Poly(A) tail (for example, RNA molecule containing some other Poly(A) tail); or the average sequence identity of multiple daughter DNA molecules obtained by replication of the parent DNA molecule to the parent DNA molecule is higher.

In this application, in the description "regulating" the expression of a RNA molecule, the "regulating" means to increase or decrease the total amount of a protein or functional RNA expressed by the RNA molecule in the same period of time, or enable the RNA to express the protein or functional RNA in a longer or shorter period of time, the increase, decrease, or longer or shorter period of time is relative to the case with another RNA molecule expressing the same protein or functional RNA. The description "regulating" a protein expression means to regulate the expression of an RNA molecule containing a coding sequence of the protein. The regulation described here can be realized by linking the Poly(A) tail of this application to the 3' end of the RNA molecule without the Poly(A) tail, or by replacing the original Poly(A) tail of the RNA with the Poly(A) tail of this application.

As used herein, percent "identity", such as 85%, 90%, 91%, 92%, 93%, 94%, 95%, 96%, 97%, 98%, 98.5%, 99%, or 99.5% identity, refers to the similarity between amino acid sequences or nucleotide sequences determined by sequence alignment, which is 85%, 90%, 91%, 92%, 93%, 94%, 95%, 96%, 97%, 98%, 98.5%, 99%, or 99.5%. For example, after two sequences are made to have the same residues in as many positions as possible by introducing gaps and other means, the ratio of the number of positions with the same base or amino acid residues to the total number of positions can be determined. The percents of "identity" can be determined by software programs known in the art. It is preferable to use the default parameters for alignment. A preferred alignment program is BLAST. Preferred programs are BLASTN and BLASTP. Details of these programs can be found at the following Internet address: ncbi.nlm.nih.gov/cgi- bin/BLAST.

As used herein, "complementarity" of nucleic acids refers to the ability of one nucleic acid to form hydrogen bonding with another nucleic acid through traditional Watson-Crick base pairing. Percent complementarity means the percentage of residues in one nucleic acid molecule that can form hydrogen bonding with another nucleic acid molecule (that is, Watson-Crick base pairing) (for example, about 5, 6, 7, 8, 9, or 10 out of 10 residues means about 50%, 60%, 70%, 80%, 90% and 100% complementarity respectively). "Complete complementarity" means that all the consecutive residues in the nucleic acid sequence form hydrogen bonding with the same number of consecutive residues in the second nucleic acid sequence. As used herein, "substantial complementarity" means, in a region having about 40, 50, 60, 70, 80, 100, 150, 200, 250 or more nucleotides, a level of complementarity of at least one of at least about 70%, 75%, 80%, 85%, 90%, 95%, 96%, 97%, 98%, 99% or 100%, or two nucleic acids hybridized under stringent conditions. For a single base or a single nucleotide, according to the Watson-Crick base pairing principle, when A is paired with T or U and C is paired with G or I, it is called complementarity or matching, and vice versa; and other base pairing than these is called non-complementarity. The "complementary polynucleotide sequence" of a polynucleotide sequence in this application refers to a polynucleotide sequence that is completely complementary to the polynucleotide sequence.

As used herein, a "conservative substitution variant" of a protein, polypeptide or amino acid sequence means that one or more amino acid residues undergo amino acid substitution(s) without changing the overall conformation and function of the protein or enzyme, including, but not limited to, amino acid substitution(s) in the amino acid sequence of a parent protein in a manner described by the aforementioned "conservative substitution". Therefore, the similarity of two proteins or amino acid sequences with similar functions may be different. For example, the similarity (identity) based on MEGALIGN algorithm is 70% to 99%. The "conservative substitution variant" also includes a polypeptide or enzyme with an amino acid identity of 60% or more, preferably 75% or more, further preferably 85% or more, and most preferably 90% or more, as determined by BLAST or FASTA algorithm, and having the same or substantially similar properties or functions as the natural or parent protein or enzyme.

In the context of this application, the terms "DNA" and "RNA" refer to single-stranded or double-stranded DNA or RNA molecules. Unless otherwise indicated, the terms "DNA" and "DNA molecule" refer to double-stranded DNA molecules formed of A, C, G and/or T nucleotide. The terms "RNA" and "RNA molecule" refer to single-stranded RNA molecules formed of A, C, G and/or U nucleotide. Herein, A, C, G, T and U nucleotide refer to nucleotides containing adenine, guanine, cytosine, thymine, and uracil as their respective nitrogenous bases.

The RNA molecules include coding RNA or non-coding RNA (ncRNA), such as Pre-mRNA, mature mRNA or long noncoding RNA (lncRNA).

As used herein, the "hybrid molecule of DNA and RNA" is a molecule containing a polynucleotide sequence consisting of deoxyribonucleotides and ribonucleotides. The hybrid molecule of DNA and RNA can be obtained by:
replacing one or more deoxyribonucleotides in DNA with a ribonucleotide;
replacing one or more ribonucleotides in RNA with a deoxyribonucleotide; or
by biological or chemical synthesis using deoxyribonucleotides and ribonucleotides as raw materials for de novo synthesis. It should be noted that the way to obtain hybrid molecules of DNA and RNA is not limited thereto, and hybrid molecules of DNA and RNA obtained in any way fall within the scope of "hybrid molecules of DNA and RNA" defined in this application.

As used herein, if two nucleic acid molecules are described as having "the same genetic information", it means that the two nucleic acid molecules are complementary or contain exactly the same base sequence, or one nucleic acid molecule differs from the other nucleic acid molecule only in that one or more bases in its base sequence are replaced by another base with the same biological function. The same biological function means that the base and the other base can undergo traditional Watson-Crick base pairing with the same base. For example, both thymine (T) and uracil (U) can undergo Watson-Crick base pairing with adenine, and both hypoxanthine (I) and cytosine (C) can undergo Watson-Crick base pairing with guanine (G). Therefore, any two of DNA, RNA and hybrid molecules of DNA and RNA can have the same genetic information. The term "base sequence" refers to the order of arrangement of bases in a polynucleotide molecule. It is known to those skilled in the art that unless otherwise indicated, when the base sequence or polynucleotide sequence described in this application is used to describe a DNA sequence, "T" is used to refer to thymine; and when the base sequence or polynucleotide sequence is used to describe RNA (for example, mRNA), "T" is replaced by "U" (uracil). Therefore, any DNA disclosed by a specific sequence number (SEQ ID NO) herein discloses a RNA (such as mRNA or Poly(A) tail) sequence complementary to or corresponding to the DNA, where each "T" in the DNA sequence is replaced by "U".

As used herein, the "hybrid molecule of DNA and RNA" refers to a nucleic acid molecule containing both deoxyribonucleotides and ribonucleotides. The "hybrid molecule of DNA and RNA" may be that one or more deoxyribonucleotide in a DNA molecule are replaced by a ribonucleotide, or one or more ribonucleotides in an RNA molecule are replaced by a deoxyribonucleotide.

### Poly(A) tail and use thereof

This application provides an engineered Poly(A) tail, which comprises one, two, three or more miRNA binding sites. Preferably, the engineered Poly(A) tail of the present invention comprises 1, 2, 3, 4, 5, 6, 7, 8, 9, 10 or more miRNA binding sites. The one, two or more miRNA binding sites can bind to the same or different miRNAs. In some embodiments, the Poly(A) tail provided in this application is obtained by inserting one, two, three or more miRNA binding sites into any Poly(A) tail without a miRNA binding site. In some embodiments, the Poly(A) tail provided in this application is obtained by inserting one or more miRNA binding location into any Poly(A) tail without a miRNA binding site. In some embodiments, the Poly(A) tail provided in this application is obtained by replacing one or more non-A bases or one or more elements structurally equivalent to element d in any Poly(A) tail without a miRNA binding site by one or more miRNA binding sites. In some embodiments, the Poly(A) tail provided in this application is obtained by replacing one or more non-A bases or one or more elements structurally equivalent to element d in any Poly(A) tail without a miRNA binding site by a miRNA binding location. The "element structurally equivalent to element d" refers to a polynucleotide fragment in a Poly(A) tail without a miRNA binding site that consists of any two or more consecutive nucleotides, has a non-A 5'- and 3'-terminal nucleotide, and does not contain more than three consecutive As, for example, element d in PCT Application No. PCT/CN2023/079037. The "Poly(A) tail without a miRNA binding site" can be any Poly(A) tail described in PCT/CN2023/079037, WO2022028559A1, WO2020/074642, and US10717982B2, and a Poly(A) tail containing exclusively A.

As used herein, the term "PolyA tail" or "PolyA sequence" refers to an unintermittent or uninterrupted sequence of adenylic acid residues usually located at the 3' end of a RNA molecule. In RNA, in the presence of 3'-UTR, the Poly-A sequence is linked to the 3' end of 3'-UTR. The unintermittent poly-A tail is characterized by consecutive adenylic acid residues. The Poly-A tail can be of any length. In some embodiments, the Poly-A tail comprises or consists of at least 20, at least 30, at least 40, at least 80 or at least 100 and at most 500, at most 400, at most 300, at most 200 or at most 150 adenylic acids (As), especially about 120 As. Generally, the vast majority of nucleotides in the PolyA tail are adenylic acid, where the vast majority means at least 75%, at least 80%, at least 85%, at least 90% of nucleotides; but the remaining nucleotides are allowed to be nucleotides other than A (non-A nucleotides), such as U (uridylic acid), G (guanylic acid) or C (cytidylic acid).

In some embodiments, the in-vitro preparation process of the RNA is a prokaryotic fermentation process, that is, a coding nucleic acid of a RNA molecule containing the Poly(A) tail is introduced into a prokaryotic cell, the prokaryotic cell is amplified to amplify the coding nucleic acid, and then, then the amplified coding nucleic acid is transcribed into the RNA. In some embodiments, the in vitro preparation process of the RNA is to link a RNA fragment containing the protein coding sequence to the Poly(A) tail by homologous recombination and enzymatic cleavage, or by other non-homologous recombination methods. The Poly(A) tail is produced by prokaryotic fermentation. During the prokaryotic fermentation process, a coding nucleic acid containing the Poly(A) tail is introduced into a prokaryotic cell, the prokaryotic cell is amplified to amplify the coding nucleic acid, and then, the amplified coding nucleic acid is transcribed into the RNA containing the tail RNA. In some embodiments, the coding nucleic acid is linear. In some embodiments, the coding nucleic acid is circular. In some embodiments, the coding nucleic acid is a plasmid. In some embodiments, the coding nucleic acid is single-stranded or double-stranded. In some embodiments, the coding nucleic acid is chemically modified before introduction into the prokaryotic cell. In some embodiments, the coding nucleic acid is chemically synthesized before introduction into the prokaryotic cell. In some embodiments, the coding nucleic acid is inserted into the nucleoidal genomic DNA of the prokaryotic cell. In some embodiments, the coding nucleic acid exists freely in the cytoplasm or outside the nucleoid of the prokaryotic cell. In some embodiments, the prokaryotic cell is *E. coli.*

Based on this, this application provides a series of engineered Poly(A) tail. The engineered Poly(A) tail comprises a miRNA binding site. In some embodiments, the bases of the engineered Poly(A) tail are all As except for the miRNA binding site. In some embodiments, the engineered Poly(A) tail is further highly conserved during the in-vitro preparation of RNA. In some embodiments, the engineered Poly(A) tail can improve the mRNA stability. In some embodiments, the engineered Poly(A) tail is formed by inserting the miRNA binding site between any two nucleotides of the conserved Poly(A) tail. In some embodiments, the engineered Poly(A) tail is formed by the direct linking (adjoining) of the miRNA binding site to the 5'-terminal nucleotide of the conserved Poly(A) tail at the 5' side of the conserved Poly(A) tail. In some embodiments, the engineered Poly(A) tail is formed by the direct linking (adjoining) of the miRNA binding site to the 3'-terminal nucleotide of the conserved Poly(A) tail at the 3' side of the conserved Poly(A) tail. In some embodiments, the engineered Poly(A) tail is formed by replacing any one or more elements in the conserved Poly(A) tail by one or more miRNA binding sites.

As used herein, the term "conserved Poly(A) tail" refers to a class of Poly(A) tails containing at least one non-A base, which are highly conserved in the in-vitro preparation process of RNA and/or can improve the stability of mRNA compared with Poly(A) tails with bases that are exclusively A. The conserved Poly(A) can be a natural Poly(A) tail or a synthetic Poly(A) tail, including, but not limited to, various Poly(A) tails described in PCT Application No. PCT/CN2023/079037, Chinese Patent Application No. CN112805386A, and US Patent No. US 10717982B2, which are is hereby incorporated herein in their entireties.

As used herein, two elements connected by "-" are directly connected. The "directly connected" means that there is no nucleotide between the two elements, so the "directly connected" can be connected by any connection method allowed between nucleotides. In some embodiments, the "directly connected" can be connected by a chemical bond. In some embodiments, the "directly connected" can be connected by a phosphodiester bond.

Additionally, this application also provides use of the Poly(A) tail in enabling the low expression of a target gene in a particular organ, tissue and/or cell, where the particular organ, highly expresses the miRNA.

### Engineered DNA molecule

This application also provides an engineered DNA molecule that can be replicated in cells, which comprises a coding sequence of the engineered Poly(A) tail or a complementary sequence thereof. In some embodiments, the engineered DNA molecule can be replicated in cells. In some embodiments, the engineered DNA molecule can express the engineered Poly(A) tail. In some embodiments, the engineered DNA molecule can express the engineered Poly(A) tail and be replicated in cells. In some embodiments, the cell is a prokaryotic cell or a eukaryotic cell. In some embodiments, the prokaryotic cell is *E. coli.* It is known to those skilled in the art that in addition to the coding sequence of the engineered Poly(A) tail, the engineered DNA molecule further comprises a structural element essential for the replication or efficient replication of the DNA molecule in cells. The structural element essential for the replication or efficient replication of the engineered DNA molecule in cells are known in the art, including, for example, the origin of replication (ORI). In some embodiments, the engineered DNA molecule further comprises a marker gene or a fragment thereof and/or a reporter gene or a fragment thereof, and a unique restriction enonuclease site, preferably a restriction enonuclease site in the form of a multiple cloning site (MCS), which allows for the insertion of a DNA element. The marker gene facilitates the identification of cells comprising plasmids containing the marker gene, and can be selected from, for example, antibiotic resistance genes. Each restriction endonuclease site in MCS can be specifically recognized by a different restriction endonuclease.

In some embodiments, the DNA molecule is a DNA plasmid. As used herein, the term "DNA plasmid" refers to a plasmid formed of a double-stranded DNA molecule. In some embodiments, the "plasmid" is a circular DNA molecule. In some embodiments, the "plasmid" can further cover a linear DNA molecule. Particularly, the term "plasmid" also covers a molecule obtained by linearizing a circular plasmid by cleaving the circular plasmid with a restriction endonuclease to convert the circular plasmid molecule into a linear molecule, and a linear molecule that can be replicated in prokaryotes. The plasmid can be replicated, that is, amplified in cells independently of genomic genetic information stored in the nucleoid in prokaryotic cells, and can be used for cloning, that is, for amplifying genetic information in bacterial cells. Preferably, the DNA plasmid according to the present invention is a medium-copy plasmid or a high-copy plasmid, more preferably a high-copy plasmid. Examples of such a high-copy plasmid are vectors based on pUC, pTZ plasmids or any other plasmids (e.g., pMB1, pCoIE1) containing ORI supporting high copy of the plasmids, etc.

In some embodiments, the DNA molecule is a DNA molecule or a fragment thereof that constitutes a nucleoid of a prokaryote, that is, the coding sequence containing the engineered Poly(A) tail or a complementary sequence thereof can be replicated with the genome of the prokaryote.

In some embodiments, the DNA molecule is genomic DNA, such as viral genomic DNA or eukaryotic genomic DNA. In some embodiments, the DNA is mitochondrial DNA. In some embodiments, the DNA is free DNA when introduced into a eukaryotic cell. In some embodiments, the DNA is a viral vector.

In some embodiments, the DNA molecule is further connected with a target gene fragment at the 5' side of the coding sequence of the engineered Poly(A) tail, where the target gene fragment and the coding sequence of the engineered Poly(A) tail jointly encode the RNA. In some embodiments, the target gene fragment and the coding sequence of the engineered Poly(A) tail jointly encode the mRNA. The target gene fragment comprises a coding sequence of a protein, a polypeptide or a fragment thereof. The coding sequence of the protein, polypeptide or fragment thereof can be finally translated into one or more proteins, or one or more polypeptides, such as short peptides, oligopeptides, polypeptides, fusion proteins, proteins and fragments thereof, for example, a moiety of a known protein, such as a functional moiety. The functional moiety may be, for example, a biologically active moiety of the protein or an antigenic moiety, such as an epitope, that can effectively produce an antibody. The coding sequence of the protein, polypeptide or fragment thereof respectively comprises a start codon (5' end) and a stop codon (3' end) at two ends, which are the first three nucleotides and the last three nucleotides of the mRNA molecule that can be translated.

In some embodiments, the mRNA of the present invention further comprises 5'UTR and 3'UTR, etc. In some embodiments, the mRNA of the present invention comprises at least, from 5' to 3', the 5'UTR sequence, the target gene sequence, the 3'UTR sequence, and the Poly(A) tail sequence of the present invention.

The 5'UTR usually contains at least one ribosome binding site (RBS), such as a Shine-Dalgarno sequence in a prokaryote, or at least one translation initiation site, such as a Kozak sequence in a eukaryote. The RBS promotes the effective and accurate translation of the mRNA molecule by recruiting ribosomes at the beginning of translation. The activity of a given RBS or translation initiation site can be optimized by changing its length and sequence and the distance from the start codon. Optionally, the 5'UTR includes an internal ribosome entry site or IRES. The 3'UTR can contain one or more regulatory sequences, such as a binding site of an amino acid sequence to enhance the stability of the mRNA molecule, a binding site to regulate the RNA molecule (such as a miRNA molecule), and/or a signal sequence involved in the intracellular transport of the mRNA molecule.

The coding sequence of the protein, polypeptide or fragment thereof contains codons that can be translated into an amino acid sequence. All the codons contained in the coding sequence may be naturally occurring codons encoding amino acids or may be partially or completely composed of synthetic codons. In some embodiments, some or all of the codons are codon optimized. In some embodiments, the some or all of the codons encode unnatural amino acids.

In some embodiments, the DNA molecule further comprises a structural element essential for initiating or regulating the RNA transcription at the 5' side of the target gene fragment, wherein the structural element is known in the art. In some embodiments, the structural element comprises at least a promoter. The promoter and its sequence are known in the art, including a weak promoter, a moderately strong promoter, a strong promoter, a mini promoter, or a core promoter. In some specific embodiments, the promoter is a strong promoter. In some embodiments, the promoter can initiate the transcription of the target gene fragment and/or engineered Poly(A) tail in a prokaryotic cell. In some embodiments, the promoter can initiate the transcription of the target gene fragment and/or engineered Poly(A) tail in a eukaryotic cell. The "promoter" includes at least one transcription recognition site followed by a transcription factor binding site. The recognition and binding sites can interact with an amino acid sequence that mediates or regulates transcription. Compared with the recognition site, the binding site is closer to the target gene fragment. The binding site can be, for example, a Pribnow box in a prokaryote or a TATA box in a eukaryote. For example, in some embodiments, when the Pribnow box is used, the transcription recognition site can be located about 35 bp upstream of the transcription initiation site, and the transcription factor binding site can be located about 10 bp upstream of the transcription initiation site. In some embodiments, the promoter comprises at least one additional regulatory element, such as an AT-rich upstream element located about 40 and/or 60 nucleotides before the transcription initiation site, and/or an additional regulatory element located between the recognition site and the binding site to enhance the promoter activity. In some embodiments, the promoter is a strong promoter. That is, the promoter contains a sequence that promotes the transcription of the RNA coding sequence. The strong promoter is known to those skilled in the art, such as OXB18, OXB19 and OXB20 promoters derived from RecA promoter from *E. coli,* or can be identified or synthesized by a routine laboratory procedure. In some embodiments, the promoter is a T7 promoter. In some embodiments, the promoter further includes an additional regulatory element, such as an enhancer included in the DNA plasmid that can promote the transcription of the RNA coding sequence.

Additionally, this application further provides use of the engineered DNA molecule in stably amplifying a coding sequence of the engineered Poly(A) tail or a coding sequence of RNA with the engineered Poly(A) tail.

### Engineered RNA

This application provides an engineered RNA or an engineered RNA molecule, which comprises the engineered Poly(A) tail as described above and a target gene fragment at the 5' side of the coding sequence of the Poly(A) tail. In some embodiments, the RNA is mRNA.

In some embodiments, the mRNA molecule in this application comprises 5'UTR and/or 3'UTR.

In some embodiments, the mRNA molecule in this application further comprises a 5' cap. In a preferred embodiment, the 5' cap is m7G(5')ppp(5')(2'-OMeA)pG.

In some embodiments, the mRNA molecule in this application further comprises a chemical modification, for example, all or some of uridine in the polynucleotide sequence is modified into N1- methyl pseudouridine.

As used herein, "mRNA" (messenger RNA) is any naturally occurring, non-naturally occurring or modified RNA encoding at least one protein, polypeptide or a fragment thereof. The mRNA has the ability to be translated to produce the encoded protein, polypeptide or a fragment thereof in vitro, in vivo, in situ or ex vivo. Therefore, the mRNA may be a mature mRNA or a pre-mature mRNA, and the essential or optional elements or structures therein are known in the art. In some embodiments, the mRNA contains coding sequences of multiple essential functional components, to express, regulate, or enhance the expression level of the protein, polypeptide or a fragment thereof. The functional component includes, but is not limited to, 5' hat, 5'UTR, or 3'UTR, etc. Both 5'UTR and 3'UTR are usually transcribed from the genomic DNA, and are elements of mRNA before maturation.

For a mature mRNA, the term "5' hat" is usually located at the 5' end of the mRNA, and contains a methylated guanylic acid, which is connected to the 5' end of the mRNA via pyrophosphate and forms a 5',5'-triphosphate linkage with a contiguous nucleotide. There are usually three types of 5' hat structures (m7G5'ppp5'Np, m7G5'ppp5'NmpNp, and m7G5'ppp5'NmpNmpNp), which are called type O, type I and type II respectively. Type O means that the ribose of the terminal nucleotide is non-methylated, type I means that the ribose of one terminal nucleotide is methylated, and type II means that the ribose of two terminal nucleotides is methylated. In some embodiments, the 5' hat can be produced according to the manufacturer's instruction by capping the 5' end of the polynucleotide using the following chemical RNA cap analogue during in-vitro transcription to produce a 5'-guanosine cap structure: 3'-O-Me-m7G(5')ppp(5')G[ARCA cap], G(5')ppp(5')A, G(5')ppp(5')G, m7G(5')ppp(5')A, m7G(5')ppp(5')G (NewEnglandBioLabs, Ipswich, MA), or m7G(5')ppp(5')(2'-OMeA)pG (CleanCapAG). For example, in some embodiments, the 5' capping of the modified RNA can be completed after transcription using a vaccinia capping enzyme to produce the type O hat structure: m7G(5')ppp(5')G (New England BioLabs, Ipswich, MA). The vaccinia capping enzyme and 2'-O-methyltransferase- can be used to produce the type I cap structure, that is, m7G(5')ppp(5')(2'-OMeA)pG. The type II hat structure can then be produced from the type I hat structure by 2'-O-methylation of the third nucleotide from the 5'-end using the 2'-O-methyltransferase. The type III hat structure can then be produced from the type II hat structure by 2'-O-methylation of the fourth nucleotide from the 5'- end using 2'-O-methyltransferase.

In some embodiments, the mRNA further comprises a stabilizing element. The stabilizing element may comprise, for example, a histone stem loop. In some embodiments, the mRNA comprises a coding region, at least one histone stem loop and optionally a poly(A) sequence or polyadenylation signal. The poly(A) sequence or polyadenylation signal generally enhances the expression level of the encoded protein. In some embodiments, the mRNA comprises a combination of the poly(A) sequence or the polyadenylation signal with at least one histone stem loop. Although the two have a substitution mechanism in nature, their synergistic effect can increase the protein expression to a level higher than that observed with any single element. The synergistic effect of the combination of poly(A) and at least one histone stem loop does not depend on the order of elements or the length of the poly(A) sequence. In some embodiments, the histone stem loop is usually derived from a histone gene, and includes intramolecular base pairing of two contiguous moieties or completely reversely complementary sequences separated by a spacer (formed of a short sequence) to form a loop. The non-paired loop region is usually unable to base pair with any of the stem-loop elements. The stability of the stem loop structure usually depends on the length, the number of mispairing or protrusions and the base composition of the paired region. In some embodiments, wobble base pairing (non-Watson-Crick base pairing) may occur. In some embodiments, the at least one histone stem loop sequence comprises 15 to 45 nucleotides in length.

In some embodiments, one or more AU-rich sequences of the mRNA can be removed. These sequences are sometimes called AURES, which are destabilizing sequences found in 3'UTR. AURES can be removed from the mRNA. Alternatively, AURES can be retained in mRNA.

### Cells

This application further provides a cell containing the engineered DNA molecule, where the DNA molecule can be stored and/or amplified in the cell. In some embodiments, the cell is a prokaryotic cell, and the DNA molecule can be replicated in the prokaryotic cell. In some embodiments, the cell is a prokaryotic cell, and the DNA molecule can be replicated and/or transcribed in the prokaryotic cell. In some embodiments, the DNA molecule is a eukaryotic cell, and the DNA molecule can be replicated in the cell. In some embodiments, the DNA molecule can be transcribed and/or replicated in the cell.

In some embodiments, the cell is a prokaryotic cell. In some embodiments, the cell is a bacterium, actinomycete, cyanobacterium, mycoplasma, rickettsia and chlamydia. In some embodiments, the cell is selected from *Bacillus subtilis,* Lactobacillus, Acetobacter, Corynebacterium, Brevibacterium, Arthrobacter, Pseudomonas, and Micrococcus. In some embodiments, the cell is a recA⁻ expressing bacterium. In some embodiments, the cell is *E*. *coli.* In some embodiments, the cell is *E. coli* selected from: K-12 and its derivative strains, and B strain and its derivative strains. In some embodiments, the *E. coli* is selected from MG1655, DH5 or DH5α, DH10B, BL21, DB3.1, HB101, JM109, JM110, MC1061, MG1655, Pir1, Stbl2, Stb13, Top10, XL1Blue, XL10 Gold, BLR, HMS174, Tuner, Rostetta2, Lemo21, T7Express, or Origami2. In some embodiments, the cell is selected from Streptomyces, Micromonospora and Nocardia. In some embodiments, the cell is a fungus. In some embodiments, the cell is selected from yeasts or molds.

### Lipid nanoparticles

This application further provides a lipid nanoparticle containing the engineered Poly(A) tail, the engineered RNA molecule, and/or the engineered DNA molecule according to this application.

In some embodiments, lipid is mixed with the engineered RNA to form a lipid nanoparticle. In some embodiments, the engineered Poly(A) tail, the engineered RNA molecule, and/or the engineered DNA molecule according to this application is formulated in the lipid nanoparticle. In some embodiments, for the lipid nanoparticle, an empty lipid nanoparticle is formed firstly, and then combined or encapsulated with the engineered Poly(A) tail, the engineered RNA molecule, and/or the engineered DNA molecule immediately before application (for example, within a few minutes to an hour).

The lipid nanoparticle usually contains an ionizable lipid, a non-cationic lipid, a sterol, a PEGylated lipid component and a target nucleic acid, such as the engineered Poly(A) tail, the engineered RNA molecule, and/or the engineered DNA molecule according to this application.

The lipid nanoparticle of this application contains an ionizable lipid, a phospholipid, a structural lipid and a PEGylated lipid. Preferably, the molar ratio of the ionizable lipid, the phospholipid, the structural lipid and the PEGylated lipid is: (20-60): (5-25): (25-55): (0.5-15), and further preferably (40-55): (5-15): (30-50): (1-3);

The phospholipid is preferably one or more selected from the following compounds:
dilauroyl-sn-glycero-phosphocholine (DLPC),
dimyristoyl-sn-glycero-phosphocholine (DMPC),
dioleoyl-sn-glycero-phosphocholine (DOPC),
dipalmitoylphosphatidylcholine (DPPC),
distearoyl-sn-glycero-3-phosphocholine (DSPC),
diundecanoyl-sn-glycero-phosphocholine (DUPC),
palmitoyl-oleoyl-glycero-phosphocholine (POPC),
1,2-di-O-octadecyl-sn-glycero-3-phosphocholine (18:0 Diether PC),
1-oleoyl-2-cholesterylhemisuccinoyl-sn-glycero-3-phosphocholine (OChemsPC),
1-hexadecyl-sn-glycero-3-phosphocholine (C16 Lyso PC),
1,2-divinyl-sn-glycero-3-phosphocholine,
1,2-diarylacyl-sn-glycero-3-phosphocholine,
1,2-dioleoyl-SN-glycero-3-phosphoethanolamine (DOPE),
1,2-distearoyl-sn-glycero-3-phosphoethanolamine,
1,2-divinyl-sn-glycero-3-phosphoethanolamine,
1,2-divinyl-sn-glycero-3-phosphoethanolamine,
1,2-diaryl-sn-glycero-3-phosphoethanolamine,
1,2-dithiohexaenoic acid-sn-glycero-3-phosphoethanolamine,
1,2-Dioleoyl-sn-glycero-3-phosphatidyl-(1-glycerol) sodium salt (DOPG) or sphingomyelin.

For example, the phospholipid is DOPE or DSPC;
the structural lipid is preferably one or two or more selected from cholesterol, coprosterol, sitosterol, ergosterol and stigmasterol, for example, the structural lipid is cholesterol; and/or
the PEGylated lipid is preferably one or more selected from PEG-modified phosphatidylethanolamine, PEG-modified phosphatidic acid, PEG-modified ceramide, PEG-modified dialkyl amine, PEG-modified diacylglycerol or PEG-modified dialkyl glycerol, for example, the PEGylated lipid is DMG-PEG2000.

Components, compositions and methods generally known in the art can be used to produce the lipid nanoparticles of the present disclosure, see, for example, PCT/US2016/052352, PCT/US2016/068300, PCT/US2017/037551, PCT/US2015/027400, PCT/US2016/047406, PCT/US2016000129, PCT/US2016/014280, PCT/US2016/014280, PCT/US2017/038426, PCT/US2014/027077, PCT/US2014/055394, PCT/US2016/52117, PCT/US2012/069610, PCT/US2017/027492, PCT/US2016/059575 and PCT/US2016/069491, which are incorporated herein by reference in their entireties.

It is to be understood that this application includes various aspects, embodiments and combinations of such aspects and/or embodiments described herein. The above description and the following examples are intended to illustrate rather than limit the scope of this application. Other aspects, improvements and modifications within the scope of this application will be obvious to those skilled in the art to which this application belongs. Therefore, those of ordinary skill in the art will recognize that the described improvements and modifications to the described aspects and embodiments are also embraced in the scope of this application.

### Examples

### Example 1: Construction of luciferase mRNA containing polyA variant of the present invention

### 1) Construction of a vector containing a luciferase protein coding region

In the vector, the E. coli cloning vector pUC57 was used as a vector backbone, and a T7 promoter sequence (SEQ ID NO: 2), 5'UTR, a Kozak sequence (GCCACC), a luciferase protein coding sequence, 3'UTR and poly(dA:dT) designated as RG008 were arranged in sequence between Xba I restriction site and SapI restriction site of its multiple cloning sites and used as a control of the poly(A) variant designed in the present invention.

### 2) Replacement of poly(dA:dT) in the universal vector

All the sequences needed to construct different poly(A) variants (as shown in the sequence list at the end of the disclosure) were synthesized. The variant poly(dA:dT) designated as RG008 was removed from the universal vector constructed in 1) by enzymatic digestion with two restriction endonucleases, and then different poly(A) variants of the present invention were respectively ligated by T4 DNA ligase 1 to the vector from which poly(dA:dT) was removed, to complete the replacement of poly(dA:dT) in the univeral vector.

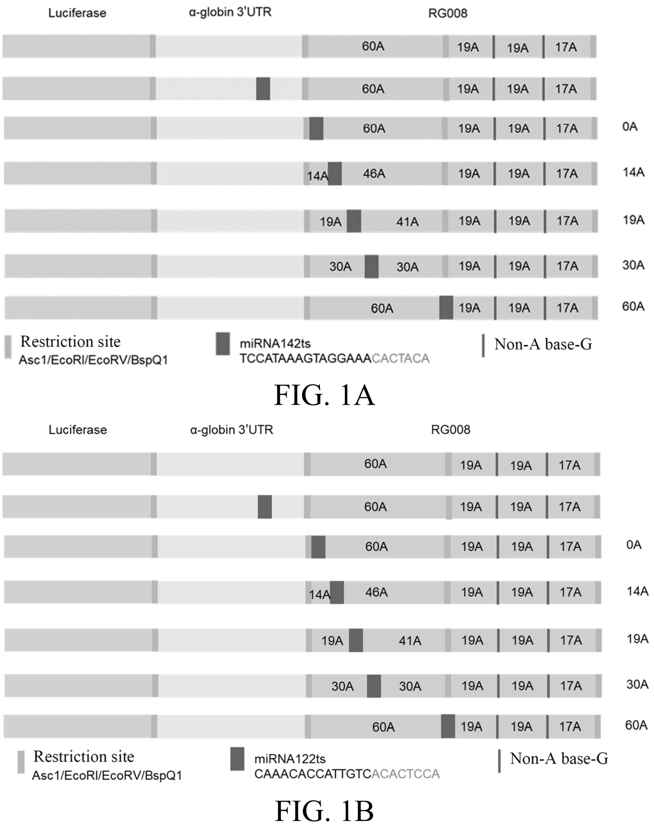

Schematic diagrams are shown in FIGs. 1A and 1B.

### Example 2: Preparation of mRNA-LNP of the present invention

An acetic acid solution was added to a luciferase mRNA stock solution until the final concentration of acetic acid was 20 mmol/L and the final concentration of mRNA was 200 µg/ml, and then stirred and mixed evenly to obtain a mRNA working solution. The mRNA working solution was mixed with a mixed lipid solution (prepared according to Table 1) by a T-shaped mixing device at a flow rate of 2:1-4:1, to prepare LNP. Then, LNP was 2-5-fold diluted with 2 mmol/L acetic acid solution, and then replaced 3 or more times with 2 mmol/L acetic acid solution. The solution was concentrated to a target concentration. A sucrose solution was added to adjust the osmotic pressure and the pH was adjusted to 7.0-8.0 with a Tris solution, to obtain the mRNA-lipid nanoparticles (LNP) of the present invention.

**Table 1: Composition of mixed lipid solution**

| Mixed lipid components | mol% |
|---|---|
| Ionizable lipid | 40-55 |
| Cholesterol | 30-50 |
| Phospholipid | 5-15 |
| DMG-PEG2000 | 1-3 |

### Example 3. Test of expression of luciferase mRNA with 5 Poly(A) containing a miR-142 binding site in mice

With luciferase coding region as a protein coding region, the expression levels of 5 Poly(A) variants (Poly(A)-60A insert miR-142; Poly(A)-30A insert miR-142; Poly(A)-19A insert miR-142; Poly(A)-14A insert miR-142; and Poly(A)-0A insert miR-142) in the liver of mice with low expression of miR-142 and the spleen of mice with high expression of miR-142 were investigated, to detect whether the insertion of miR-142 binding site can down-regulate the expression level of mRNA in organs with high expression of miR-142.

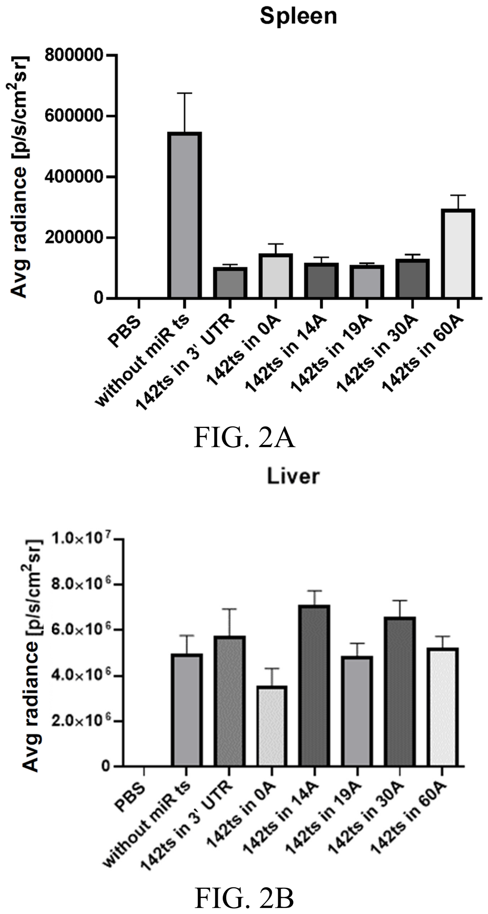

C57BL/6 mice were randomly grouped according to their body weight, and 6 animals in each group were raised adaptively for 2-3 days. The mice were administered by injection via tail vein at a dosage of 0.05 mg/kg of mRNA-LNPs (1 µg) in an injection volume of 100 µL. The animals in the blank control was injected with PBS of the same volume. 6 h after administration, Luciferin (200 µL) was injected intraperitoneally at a dose of 150 mg/kg. After 10 min, the mice were anesthetized, and imaged with a small animal imager. The liver and spleen of the mice were dissected and imaged again. The experiment was conducted once, and a histogram was drawn with the measured values of each mouse. The results are shown in Figs. 2A-2B.

Conclusions: Various Poly(A) variants are formed by inserting the miR-142 binding site at a position after 0A (SEQ ID NO: 16), 14A (SEQ ID NO:9), 19A (SEQ ID NO: 8), 30A (SEQ ID NO: 7), 60A (SEQ ID NO: 6) of RG008. When mRNAs with such variants are packaged with LNPs, and injected into mice through the tail vein, the mRNAs will be uptaken and degraded in the spleen enriched with macrophages (highly expressing miR-142), and their expression will be inhibited. In terms of the expression level in spleen, the decrease in expression is comparable when the miR-142 binding site is inserted in 3'UTR or at a position after 0A, 14A, 19A, and 30A, and is significantly better than the case when it is inserted after 60A.

In terms of the expression level in liver, when the miR-142 binding site is inserted at a position after 14A, 19A, 30A, and 60A, the expression level in liver is basically unaffected; when it is inserted at a position after 14A and 30A, the expression level is even better than that in the control group without the miR-142 binding site; and when it is inserted at a position after 0A, the expression level in liver decreases obviously.

Based on the expression levels in the liver and spleen, the insertion positions 14A-30A are screened out and used as the optimal insertion positions of the miR-142 binding site in the present invention, with which the expression level in spleen can be significantly reduced while the expression level in liver is not affected. Therefore, the present application is suitable for the application scenario for treating diseases with mRNAs expressing proteins in the liver.

### Example 4. Test of expression of luciferase mRNA with 5 Poly(A) containing a miR-122 binding site in mice

The expression levels of 5 Poly(A) variants (Poly(A)-60A insert miR-122; Poly(A)-30A insert miR-122; Poly(A)-19A insert miR-122; Poly(A)-14A insert miR-122; Poly(A)-0A insert miR-122) in the spleen of mice with low expression of miR-122 and the liver of mice with high expression of miR-122 were investigated.

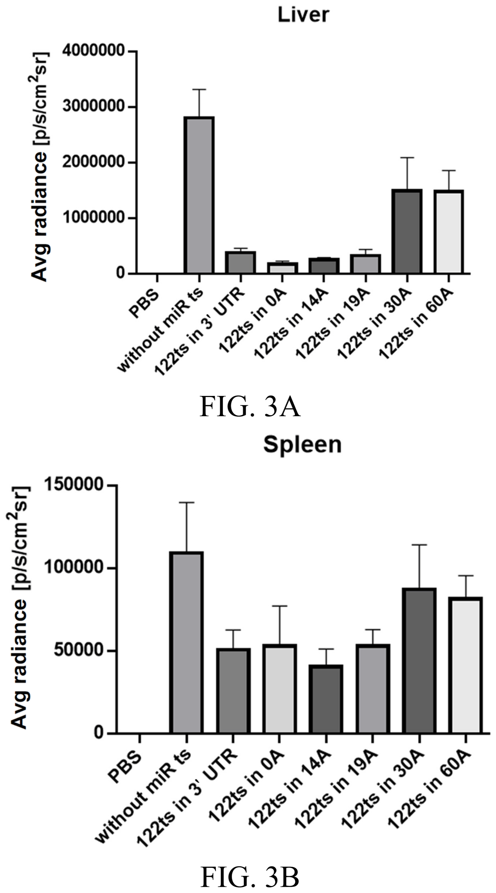

C57BL/6 mice were randomly grouped according to their body weight, and 6 animals in each group were raised adaptively for 2-3 days. The mice were administered by injection via tail vein at a dosage of 0.05 mg/kg of mRNA-LNPs (1 µg) in an injection volume of 100 µL. The animals in the blank control was injected with PBS of the same volume. 6 h after administration, Luciferin (200 µL) was injected intraperitoneally at a dose of 150 mg/kg. After 10 min, the mice were anesthetized, and imaged with a small animal imager. The liver and spleen of the mice were dissected and imaged again. The experiment was conducted once, and a histogram was drawn with the measured values of each mouse. The absolute values of fluorescence imaging of the liver and spleen are shown in Figs. 3A-3B, respectively.

Conclusions: Various Poly(A) variants are formed by inserting the miR-122 binding site at a position after 0A, 14A, 19A, 30A, and 60A of RG008 respectively. When mRNAs with such variants are packaged with LNPs, and injected into mice through the tail vein, the mRNAs are uptaken and degraded obviously in the liver, and their expression will be inhibited. When the miR-122 binding site is inserted at a position after 0A, 14A, and 19A of RG008, the decrease in expression in the liver is significantly lower than the case when it is inserted at a position after 30A and 60A. The expression level in spleen decreases in all groups. Based on the expression levels in the liver and spleen, the insertion positions 0A, 14A, and 19A are screened out and used as the optimal insertion positions of the miR-122 binding site in the present invention, with which the expression level in spleen maintain while the expression level in liver is not significantly affected. Therefore, the present application is suitable for the application scenario for treating diseases with mRNAs expressing proteins in the spleen.

### Example 5. Test of stability of plasmids containing different poly(A) variants in replication in E. coli

The effects of a total of 10 Poly(A) variants (Poly(A)-60A insert miR-142; Poly(A)-30A insert miR-142; Poly(A)-19A insert miR-142; Poly(A)-14A insert miR-142; Poly(A)-0A insert miR-142; Poly(A)-60A insert miR-122; Poly(A)-30A insert miR-122; Poly(A)-19A insert miR-122; Poly(A)-14A insert miR-122; and Poly(A)-0A insert miR-122) in two groups on the stability in E. coli were investigated.

After the vector plasmid constructed according to the method in Example 1 was confirmed to be correct by sequencing, it was transformed into *E. coli* DH5α. The cells were grown in a plate at 30°C after transformation. The next day, the plate was sent to a sequencing service company and 50 clones were randomly selected, to complete the sequencing work. After sequencing, the different Poly(A) variants were analyzed and calculated according to the sequencing results.

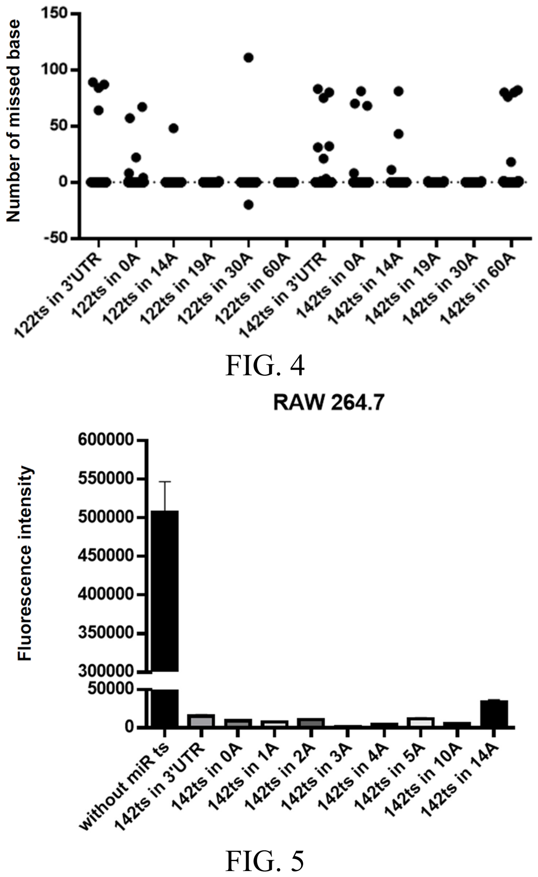

The results are shown in Fig. 4. The rates of base deletion after two different miRNA binding sites are inserted after positions 14A-30A of the Poly(A) variants are lower than the case when they are inserted at positions after 0A or 60A and into 3'UTR. The results show that the plasmids with the miRNA binding site inserted after 14A-30A in RG008 Poly(A) variants are more stable.

### Example 6. Test of expression of mRNAs with Poly(A) variants containing a miR-142 binding site at different insertion positions in cells

The expression levels of 8 Poly(A) variants (Poly(A)-0A insert miR-142; Poly(A)-1A insert miR-142; Poly(A)-2A insert miR-142; Poly(A)-3A insert miR-142; Poly(A)-4A insert miR-142; Poly(A)-5A insert miR-142; Poly(A)-10A insert miR-142; Poly(A)-14A insert miR-142) in mouse monocyte/macrophage leukemia cells RAW 264.7 with high expression of miR-142.

The RAW 246.7 cells were cultured in a 24-well cell culture plate at a cell density of 1x10⁵cells/well. The cells in each well were transfected with 500 ng of mRNA synthesized in vitro in the presence of Lipofectamine 3000 (purchased from Thermo Fisher Scientific). 24 h after transfection, the cell culture medium was aspirated off, and 200 µL of a cell lysis buffer was added to each well. After 10 min, the cell lysate was transferred to a 1.5 mL EP tube, centrifuged at 12000 rpm and 4°C for 5min. Then 20 µL of the supernatant of the lysate was transferred to a black opaque 96-well plate, and 200 µL of a fluorescein substrate was added and the value was read on a fluorescence detector. The results are shown in Fig. 5.

Conclusions: Various Poly(A) variants are formed by inserting the miR-142 binding site at a position after 0A (SEQ ID NO: 16), 1A (SEQ ID NO: 15), 2A (SEQ ID NO: 14), 3A (SEQ ID NO: 13), 4A (SEQ ID NO: 12), 5A (SEQ ID NO: 11), 10A (SEQ ID NO: 10), 14A (SEQ ID NO: 9) of RG008. When mRNAs with these variants are delivered to a cell line overexpressing miR-142, such as Raw 264.7 cells, the mRNAs will be degraded by miR-142, and their expression will be inhibited.

### Example 7. Study on the interaction of various miRNA Binding Sites in tandem in Poly(A) tail

The expression levels of 2 Poly(A) variants (Poly(A)-0A insert miR-142+miR-122; and Poly(A)-0A insert miR-122+miR-142) in mouse monocyte/macrophage leukemia cells RAW 264.7 with high expression of miR-142 were investigated, to investigate whether the insertion of the miR-122 and miR-142 binding site in tandem affects the down-regulation of mRNA expression level by the miR-142 binding site.

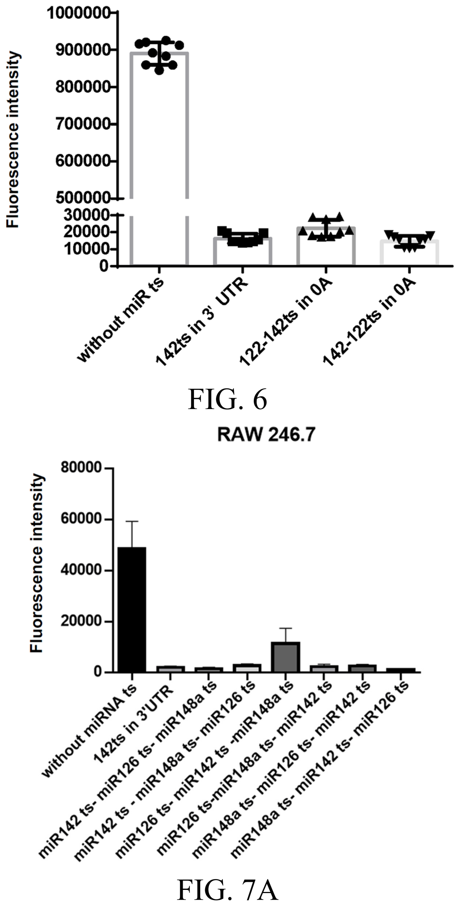

The RAW 246.7 cells were cultured in a 24-well cell culture plate at a cell density of 1x10³cells/well. The cells in each well were transfected with 500 ng of mRNA synthesized in vitro in the presence of Lipofectamine 3000 (purchased from Thermo Fisher Scientific). 24 h after transfection, the cell culture medium was aspirated off, and 200 µL of a cell lysis buffer was added to each well. After 10 min, the cell lysate was transferred to a 1.5 mL EP tube, and centrifuged at 12000 rpm and 4°C for 5min, then 20 µL of the supernatant of the lysate was transferred to a black opaque 96-well plate, and 200 µL of a fluorescein substrate was added and the value was read on a fluorescence detector. The results are shown in Fig. 6.

Conclusions: Various Poly(A) variants are formed by inserting the miR-142+miR-122 or miR-122+miR-142 binding site at a position after 0A in RG008. When mRNAs with such variants are delivered to a cell line overexpressing miR-142 through LNPs, such as Raw 264.7 cells, the mRNAs will be degraded by miR-142, and their expression will be inhibited. Moreover, miR-142 and miR-122 binding sites connected in tandem in different orders do not affect the expression inhibition effect.

### Example 8. Study on accurate expression of mRNA with Poly(A) variant containing multiple miRNA binding sites in cells

The expression levels of 6 Poly(A) variants (Poly(A)-0A insert miR-142+miR-126+miR-148a; Poly(A)-0A insert miR-142+miR-148a+miR-126; Poly(A)-0A insert miR-126+miR-142+miR-148a; Poly(A)-0A insert miR-126+miR-148a+miR-142; Poly(A)-0A insert miR-148a+miR-142+miR-126; and Poly(A)-0A insert miR-148a+miR-126+miR-142) in mouse monocyte/macrophage leukemia cells RAW 264.7 highly expressing miR-142, mouse liver sinusoidal endothelial cells (LSECs) highly expressing miR-126, mouse hepatic stellate cells (HSCs) highly expressing miR-148a and hepatic parenchymal cells AML were investigated.

The RAW246.7, LSEC, HSC, and AML12 cells were cultured in a 24-well cell culture plate at a cell density of 1x10⁵cells/well. The cells in each well were transfected with 500 ng of mRNA synthesized in vitro in the presence of Lipofectamine 3000 (purchased from Thermo Fisher Scientific). 24 h after transfection, the cell culture medium was aspirated off, and 200 µL of a cell lysis buffer was added to each well. After 10 min, the cell lysate was transferred to a 1.5 mL EP tube, and centrifuged at 12000 rpm and 4°C for 5min. Then 20 µL of the supernatant of the lysate was transferred to a black opaque 96-well plate, and 200 µL of a fluorescein substrate was added and the value was read on a fluorescence detector. Calculation of reduction rate relative to the expression in hepatocytes: The data of ts group without miRNA was defined as 1, and the relative expression levels of various groups relative to ts without miRNA in various cell line experiments were calculated, to bridge the direct expression data of various cell lines. On this basis, the rate of decreased expression in each target cell (RAW 264.7 cell, hepatic sinusoidal endothelial cell LSEC, and hepatic stellate cell HSC) relative to hepatic parenchymal cells AML12 was calculated by the formula below: (1- relative expression level in target cell/relative expression level in hepatic parenchymal cells) *100%.

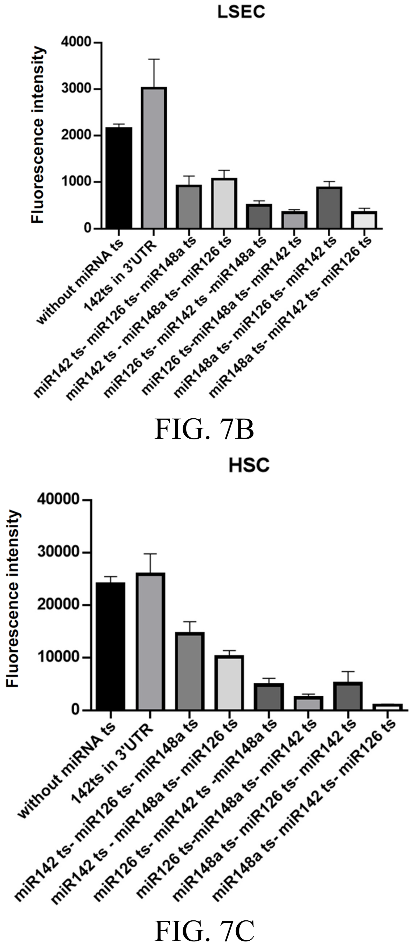

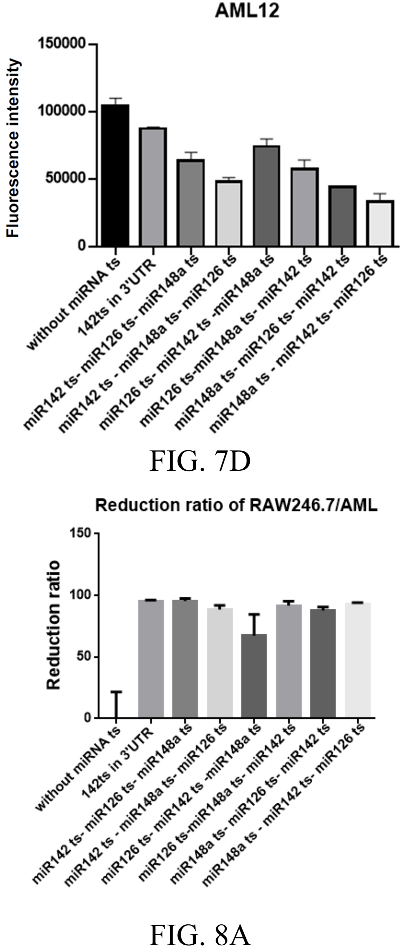

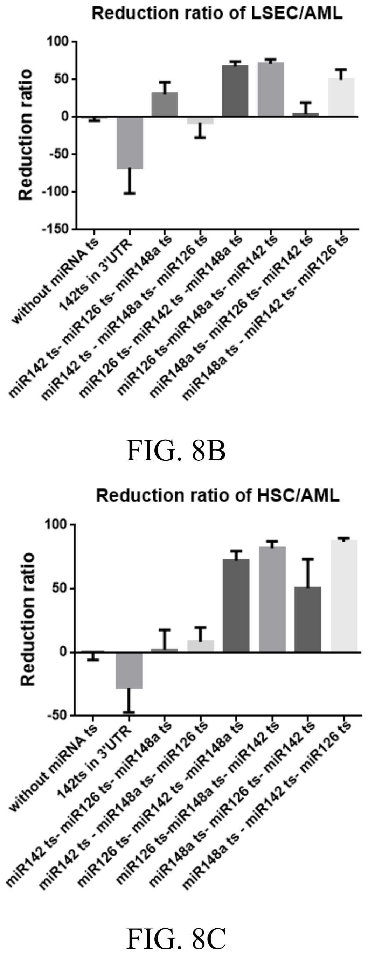

Conclusions: When miR-148a, miR-142, and miR-126 are connected in tandem and the mRNA is transfected into RAW 264.7 cells, the Poly(A) variant contains the miR-142 binding site, and the expression of the mRNA in the cell line decreases. When the mRNA is transfected into LSEC cells, the Poly(A) variant contains the miR-126 binding site, and the expression of the mRNA in the cell line decreases. When the mRNA is transfected into HSC cells, the Poly(A) variant contains the miR-148a binding site, and the expression of the mRNA in the cell line decreases. Additionally, the performance of the combinations of miR-126+miR-148a+miR-142 and miR-148a+miR-142+miR-126 in the three cells is better than the combinations in other sequences. See Figs. 7A-7D and 8A-8C.

The liver is mainly composed of hepatic parenchymal cells, hepatic macrophages, hepatic sinusoidal endothelial cells, and hepatic stellate cells. In this example, the combinations of microRNA binding sites with excellent specific expression in hepatic parenchymal cells are screened out, thus providing a reliable tool for targeted therapy of hepatic parenchymal cells.

The results of the present invention show that the insertion of a miRNA binding site into a Poly(A) variant enables the mRNA to show different expressions in different cell lines and different organs of mice. Therefore, highly specific expression regulation can be achieved by inserting one or more miRNA binding sites into the same sequence.

The sequences used in the above examples of this application are shown in the following sequence list. It is to be understood that the following sequences are merely exemplary sequences in the embodiments of this application, the present application, and are not intended to limit the embodiments of this application in any way. The nucleic acid sequence in the following sequence list represents a DNA sequence or an RNA sequence. When it represents an RNA sequence, "T" represents uridine. In addition, in the context of RNA, a single T in this application also refers to uracil or uridine.

### Sequence list:

| SEQ ID NO. | Name | Sequence |
|---|---|---|
| Nucleic acid sequence | | |
| 1 | RG008 | AAAAAAAAAAAAAAAAAAAAAAAAAAAAAAAAAAAAAAAAAAAAAAAAA AAAAAAAAAAAAAAAAAAGATATCAAAAAAAAAAAAAAAAAAAAAAGAA AAAAAAAAAAAAAAAAAAAGAAAAAAAAAAAAAAAAAAA |
| 2 | T7 promoter | TAATACGACTCACTATAAGG |
| 3 | 3'UTR | GCTGGAGCCTCGGTGGCCATGCTTCTTGCCCCTTGGGCCTCCCCCCA GCCCCTCCTCCCCTTCCTGCACCCGTACCCCCGTGGTCTTTGAATAA AGTCTGAGTGGGCGGC |
| 4 | miR-142 binding site | TCCATAAAGTAGGAAACACTACA |
| 5 | 3'UTR inserted with miR-142 binding site | GCTGGAGCCTCGGTGGCCATGCTTCTTGCCCCTTGGGCCTCCCCCCA GCCCCTCCTCCCCTTCCTGCACCCGTACCCCCTCCATAAAGTAGGAA ACACTACAGTGGTCTTTGAATAAAGTCTGAGTGGGCGGC |
| 6 | Poly(A)-60A insert miR-142 | AAAAAAAAAAAAAAAAAAAAAAAAAAAAAAAAAAAAAAAAAAAA AAAAAAAAAAAAAAAATCCATAAAGTAGGAAACACTACAAAAAAA AAAAAAAAAAAAAGAAAAAAAAAAAAAAAAAAAGAAAAAAAAAAA AAAAAAA |
| 7 | Poly(A)-30A insert miR-142 | AAAAAAAAAAAAAAAAAAAAAAAAAAAAAATCCATAAAGTAGGA AACACTACAAAAAAAAAAAAAAAAAAAAAAAAAAAAAAAGATAT CAAAAAAAAAAAAAAAAAAAGAAAAAAAAAAAAAAAAAAAGAAA AAAAAAAAAAAAAA |
| 8 | Poly(A)-19A insert miR-142 | AAAAAAAAAAAAAAAAAAATCCATAAAGTAGGAAACACTACAAAA AAAAAAAAAAAAAAAAAAAAAAAAAAAAAAAAAAAAAAGATATC |
| | | AAAAAAAAAAAAAAAAAAAAAGAAAAAAAAAAAAAAAAAAAAGAAAA AAAAAAAAAAAAAA |
| 9 | Poly(A)-14A insert miR-142 | AAAAAAAAAAAAAAATCCATAAAGTAGGAAACACTACAAAAAAAA AAAAAAAAAAAAAAAAAAAAAAAAAAAAAAAAAAAAAAAAAGATATC AAAAAAAAAAAAAAAAAAAAGAAAAAAAAAAAAAAAAAAAAAGAAAA AAAAAAAAAAAAAA |
| 10 | Poly(A)-10A insert miR-142 | AAAAAAAAAAATCCATAAAGTAGGAAACACTACAAAAAAAAAAAAAAA AAAAAAAAAAAAAAAAAAAAAAAAAAAAAAAAAAAAAAAAGATATC AAAAAAAAAAAAAAAAAAAAGAAAAAAAAAAAAAAAAAAAGAAAA AAAAAAAAAAAAAA |
| 11 | Poly(A)-5A insert miR-142 | AAAAATCCATAAAGTAGGAAACACTACAAAAAAAAAAAAAAAAAAAAA AAAAAAAAAAAAAAAAAAAAAAAAAAAAAAAAAAAAAAAAAGATATC AAAAAAAAAAAAAAAAAAAAAGAAAAAAAAAAAAAAAAAAAAGAAAA AAAAAAAAAAAAAA |
| 12 | Poly(A)-4A insert miR-142 | AAAATCCATAAAGTAGGAAACACTACAAAAAAAAAAAAAAAAAAAA AAAAAAAAAAAAAAAAAAAAAAAAAAAAAAAAAAAAAAAAAGATATC AAAAAAAAAAAAAAAAAAAGAAAAAAAAAAAAAAAAAAAGAAAA AAAAAAAAAAAAAA |
| 13 | Poly(A)-3A insert miR-142 | AAATCCATAAAGTAGGAAACACTACAAAAAAAAAAAAAAAAAAAAA AAAAAAAAAAAAAAAAAAAAAAAAAAAAAAAAAAAAAAAAAAGATATC AAAAAAAAAAAAAAAAAAAAGAAAAAAAAAAAAAAAAAAAAGAAAA AAAAAAAAAAAAAA |
| 14 | Poly(A)-2A insert miR-142 | AATCCATAAAGTAGGAAACACTACAAAAAAAAAAAAAAAAAAAAAA AAAAAAAAAAAAAAAAAAAAAAAAAAAAAAAAAAAAAAAAAGATATC AAAAAAAAAAAAAAAAAAAAGAAAAAAAAAAAAAAAAAAAAAGAAAA AAAAAAAAAAAAAA |
| 15 | Poly(A)-1A insert miR-142 | ATCCATAAAGTAGGAAACACTACAAAAAAAAAAAAAAAAAAAAAAAAA AAAAAAAAAAAAAAAAAAAAAAAAAAAAAAAAAAAAAAAAAAGATATC AAAAAAAAAAAAAAAAAAAAAGAAAAAAAAAAAAAAAAAAAAAGAAAA AAAAAAAAAAAAAA |
| 16 | Poly(A)-0A insert miR-142 | TCCATAAAGTAGGAAACACTACAAAAAAAAAAAAAAAAAAAAAAA AAAAAAAAAAAAAAAAAAAAAAAAAAAAAAAAAAAAAAAAAGATATC AAAAAAAAAAAAAAAAAAAGAAAAAAAAAAAAAAAAAAAGAAAA AAAAAAAAAAAAAA |
| 17 | miR-122 binding site | **CAAACACCATTGTCACACTCCA** |
| 18 | 3'UTR inserted with miR-122 binding site | GCTGGAGCCTCGGTGGCCATGCTTCTTGCCCCTTGGGCCTCCCCCCA GCCCCTCCTCCCCTTCCTGCACCCGTACCCCCCAAACACCATTGTC ACACTCCAGTGGTCTTTGAATAAAGTCTGAGTGGGCGGC |
| 19 | Poly(A)-60A insert miR-122 | AAAAAAAAAAAAAAAAAAAAAAAAAAAAAAAAAAAAAAAAAAAAA AAAAAAAAAAAAAAAAACAAACACCATTGTCACACTCCAAAAAAA AAAAAAAAAAAAAGAAAAAAAAAAAAAAAAAAAGAAAAAAAAA AAAAAAA |
| 20 | Poly(A)-30A insert miR-122 | AAAAAAAAAAAAAAAAAAAAAAAAAAAAAAAACAAACACCATTGTC ACACTCCAAAAAAAAAAAAAAAAAAAAAAAAAAAAAAAAAGATATC AAAAAAAAAAAAAAAAAAAAGAAAAAAAAAAAAAAAAAAAAGAAAA AAAAAAAAAAAAAA |
| 21 | Poly(A)-19A insert miR-122 | AAAAAAAAAAAAAAAAAAAAAACAAACACCATTGTCACACTCCAAAA AAAAAAAAAAAAAAAAAAAAAAAAAAAAAAAAAAAAAAAAAAGATATC AAAAAAAAAAAAAAAAAAAAAAGAAAAAAAAAAAAAAAAAAAAAAGAAAA |
| | | AAAAAAAAAAAAAA |
| 22 | Poly(A)-14A insert miR-122 | AAAAAAAAAAAAAAACAAACACCATTGTCACACTCCAAAAAAAAA AAAAAAAAAAAAAAAAAAAAAAAAAAAAAAAAAAAAAAAAAGATATC AAAAAAAAAAAAAAAAAAAAGAAAAAAAAAAAAAAAAAAAAAAGAAAA AAAAAAAAAAAAAA |
| 23 | Poly(A)-10A insert miR-122 | AAAAAAAAAAACAAACACCATTGTCACACTCCAAAAAAAAAAAAA AAAAAAAAAAAAAAAAAAAAAAAAAAAAAAAAAAAAAAAAGATATC AAAAAAAAAAAAAAAAAAAAGAAAAAAAAAAAAAAAAAAAAGAAAA AAAAAAAAAAAAAA |
| 24 | Poly(A)-5A insert miR-122 | AAAAACAAACACCATTGTCACACTCCAAAAAAAAAAAAAAAAAAA AAAAAAAAAAAAAAAAAAAAAAAAAAAAAAAAAAAAAAAAAGATATC AAAAAAAAAAAAAAAAAAAAAGAAAAAAAAAAAAAAAAAAAAGAAAA AAAAAAAAAAAAAA |
| 25 | Poly(A)-4A insert miR-122 | AAAACAAACACCATTGTCACACTCCAAAAAAAAAAAAAAAAAAAAA AAAAAAAAAAAAAAAAAAAAAAAAAAAAAAAAAAAAAAAAGATATC AAAAAAAAAAAAAAAAAAAGAAAAAAAAAAAAAAAAAAAGAAAA AAAAAAAAAAAAAA |
| 26 | Poly(A)-3A insert miR-122 | AAACAAACACCATTGTCACACTCCAAAAAAAAAAAAAAAAAA AAAAAAAAAAAAAAAAAAAAAAAAAAAAAAAAAAAAAAAAAGATATC AAAAAAAAAAAAAAAAAAAAGAAAAAAAAAAAAAAAAAAAAGAAAA AAAAAAAAAAAAAA |
| 27 | Poly(A)-2A insert miR-122 | AACAAACACCATTGTCACACTCCAAAAAAAAAAAAAAAAAAAAAA AAAAAAAAAAAAAAAAAAAAAAAAAAAAAAAAAAAAAAAAAGATATC AAAAAAAAAAAAAAAAAAAAGAAAAAAAAAAAAAAAAAAAAGAAAA AAAAAAAAAAAAAA |
| 28 | Poly(A)-1A insert miR-122 | ACAAACACCATTGTCACACTCCAAAAAAAAAAAAAAAAAAAAAAAA AAAAAAAAAAAAAAAAAAAAAAAAAAAAAAAAAAAAAAAAAGATATC AAAAAAAAAAAAAAAAAAAAGAAAAAAAAAAAAAAAAAAAAGAAAA AAAAAAAAAAAAAA |
| 29 | Poly(A)-0A insert miR-122 | CAAACACCATTGTCACACTCCAAAAAAAAAAAAAAAAAAAAAAAAAAA AAAAAAAAAAAAAAAAAAAAAAAAAAAAAAAAAAAAAAAAAAGATATC AAAAAAAAAAAAAAAAAAAAAGAAAAAAAAAAAAAAAAAAAAAGAAAA AAAAAAAAAAAAAA |
| 30 | Poly(A)-0A insert miR-122+miR-142 | **CAAACACCATTGTCACACTCCA**TCCATAAAGTAGGAAACACTACA AAAAAAAAAAAAAAAAAAAAAAAAAAAAAAAAAAAAAAAAAAAA AAAAAAAAAAAAAAAAGATATCAAAAAAAAAAAAAAAAAAAGAA AAAAAAAAAAAAAAAAAGAAAAAAAAAAAAAAAA |
| 31 | Poly(A)-0A insert miR-142+miR-122 | TCCATAAAGTAGGAAACACTACACAAACACCATTGTCACACTCC AAAAAAAAAAAAAAAAAAAAAAAAAAAAAAAAAAAAAAAAAAAA AAAAAAAAAAAAAAAAAGATATCAAAAAAAAAAAAAAAAAAAGAA AAAAAAAAAAAAAAAAAGAAAAAAAAAAAAAAAAA |
| 32 | miR-126 binding site | *CGCATTATTACTCACGGTACGA* |
| 33 | Poly(A)-60A insert miR-126 | AAAAAAAAAAAAAAAAAAAAAAAAAAAAAAAAAAAAAAAAAAAA AAAAAAAAAAAAAAAAACGCATTATTACTCACGGTACGAAAAAAAAAA AAAAAAAAAAGAAAAAAAAAAAAAAAAAAAGAAAAAAAAAAAA AAAAA |
| 34 | Poly(A)-30A insert miR-126 | AAAAAAAAAAAAAAAAAAAAAAAAAAAAACGCATTATTACTCACG GTACGAAAAAAAAAAAAAAAAAAAAAAAAAAAAAAAAAAGATATCAAA AAAAAAAAAAAAAAAGAAAAAAAAAAAAAAAAAGAAAAAAA |
| | | AAAAAAAAAA |
| 35 | Poly(A)-19A insert miR-126 | AAAAAAAAAAAAAAAAAAAAACGCATTATTACTCACGGTACGAAAAAA AAAAAAAAAAAAAAAAAAAAAAAAAAAAAAAAAAAAAAAGATATCAA AAAAAAAAAAAAAAAAAAGAAAAAAAAAAAAAAAAAAAGAAAAAA AAAAAAAAAAAA |
| 36 | Poly(A)-14A insert miR-126 | AAAAAAAAAAAAAACGCATTATTACTCACGGTACGAAAAAAAAAAA AAAAAAAAAAAAAAAAAAAAAAAAAAAAAAAAAAAAAAAAGATATCAA AAAAAAAAAAAAAAAAAAGAAAAAAAAAAAAAAAAAAAAGAAAAAA AAAAAAAAAAAA |
| 37 | Poly(A)-0A insert miR-126 | *CGCATTATTACTCACGGTACGA*AAAAAAAAAAAAAAAAAAAAAAAAAA AAAAAAAAAAAAAAAAAAAAAAAAAAAAAAAAAAAAAAAAGATATCAA AAAAAAAAAAAAAAAAAAAGAAAAAAAAAAAAAAAAAAAAAGAAAAAA AAAAAAAAAAAA |
| 38 | Poly(A-120)-0A insert miR-142 | TCCATAAAGTAGGAAACACTACAAAAAAAAAAAAAAAAAAAAAAA AAAAAAAAAAAAAAAAAAAAAAAAAAAAAAAAAAAAAAAAAAAA AAAAAAAAAAAAAAAAAAAAAAAAAAAAAAAAAAAAAAAAAAAA AAAAAAAAAA |
| 39 | Poly(A-120)-0A insert miR-122 | CAAACACCATTGTCACACTCCAAAAAAAAAAAAAAAAAAAAAAAAAAA AAAAAAAAAAAAAAAAAAAAAAAAAAAAAAAAAAAAAAAAAAAA AAAAAAAAAAAAAAAAAAAAAAAAAAAAAAAAAAAAAAAAAAAA AAAAAAAAAA |
| 40 | Poly(A-120)-0A insert miR-126 | CGCATTATTACTCACGGTACGAAAAAAAAAAAAAAAAAAAAAAAAAA AAAAAAAAAAAAAAAAAAAAAAAAAAAAAAAAAAAAAAAAAAAAA AAAAAAAAAAAAAAAAAAAAAAAAAAAAAAAAAAAAAAAAAAAAA AAAAAAAA |
| 41 | Poly(A-120)-14A insert miR-142 | AAAAAAAAAAAAAATCCATAAAGTAGGAAACTACAAAAAAAAA AAAAAAAAAAAAAAAAAAAAAAAAAAAAAAAAAAAAAAAAAAAA AAAAAAAAAAAAAAAAAAAAAAAAAAAAAAAAAAAAAAAAAAAA AAAAAAAAAA |
| 42 | Poly(A-120)-14A insert miR-122 | AAAAAAAAAAAAAACAAACACCATTGTCACACTCCAAAAAAAAAA AAAAAAAAAAAAAAAAAAAAAAAAAAAAAAAAAAAAAAAAAAAA AAAAAAAAAAAAAAAAAAAAAAAAAAAAAAAAAAAAAAAAAAAA AAAAAAAAAA |
| 43 | Poly(A-120)-14A insert miR-126 | AAAAAAAAAAAAAACGCATTATTACTCACGGTACGAAAAAAAAAAAA AAAAAAAAAAAAAAAAAAAAAAAAAAAAAAAAAAAAAAAAAAAA AAAAAAAAAAAAAAAAAAAAAAAAAAAAAAAAAAAAAAAAAAAA AAAAAAAA |
| 44 | Poly(A-120)-19A insert miR-142 | AAAAAAAAAAAAAAAAAAATCCATAAAGTAGGAAACACTACAAAA AAAAAAAAAAAAAAAAAAAAAAAAAAAAAAAAAAAAAAAAAAAA AAAAAAAAAAAAAAAAAAAAAAAAAAAAAAAAAAAAAAAAAAAA AAAAAAAAAA |
| 45 | Poly(A-120)-19A insert miR-122 | AAAAAAAAAAAAAAAAAAA**CAAACACCATTGTCACACTCC**AAAA AAAAAAAAAAAAAAAAAAAAAAAAAAAAAAAAAAAAAAAAAAA AAAAAAAAAAAAAAAAAAAAAAAAAAAAAAAAAAAAAAAAAAA AAAAAAAAAA |
| 46 | Poly(A-120)-19A insert miR-126 | AAAAAAAAAAAAAAAAAAA*CGCATTATTACTCACGGTACGA*AAAAA AAAAAAAAAAAAAAAAAAAAAAAAAAAAAAAAAAAAAAAAAA AAAAAAAAAAAAAAAAAAAAAAAAAAAAAAAAAAAAAAAAAA AAAAAAAA |
| 47 | Poly(A-120)-30A | AAAAAAAAAAAAAAAAAAAAAAAAAAAAAATCCATAAAGTAGGA |
| | insert miR-142 | AACACTACAAAAAAAAAAAAAAAAAAAAAAAAAAAAAAAAAAAAAAAAAAAA AAAAAAAAAAAAAAAAAAAAAAAAAAAAAAAAAAAAAAAAAAAA AAAAAAAAAAA |
| 48 | Poly(A-120)-30A insert miR-122 | AAAAAAAAAAAAAAAAAAAAAAAAAAAAACAAACACCATTGTC ACACTCCAAAAAAAAAAAAAAAAAAAAAAAAAAAAAAAAAAAAAAAAAA AAAAAAAAAAAAAAAAAAAAAAAAAAAAAAAAAAAAAAAAAAAA AAAAAAAAAA |
| 49 | Poly(A-120)-30A insert miR-126 | AAAAAAAAAAAAAAAAAAAAAAAAAAAAACGCATTATTACTCACG GTACGAAAAAAAAAAAAAAAAAAAAAAAAAAAAAAAAAAAAAAAAA AAAAAAAAAAAAAAAAAAAAAAAAAAAAAAAAAAAAAAAAAAA AAAAAAAA |
| 50 | Poly(A-120)-60A insert miR-142 | AAAAAAAAAAAAAAAAAAAAAAAAAAAAAAAAAAAAAAAAAAA AAAAAAAAAAAAAAAATCCATAAAGTAGGAAACACTACAAAAAAA AAAAAAAAAAAAAAAAAAAAAAAAAAAAAAAAAAAAAAAAAAA AAAAAAAAAA |
| 51 | Poly(A-120)-60A insert miR-122 | AAAAAAAAAAAAAAAAAAAAAAAAAAAAAAAAAAAAAAAAAAAA AAAAAAAAAAAAAAAACAAACACCATTGTCACACTCCAAAAAAA AAAAAAAAAAAAAAAAAAAAAAAAAAAAAAAAAAAAAAAAAAAA AAAAAAAAAA |
| 52 | Poly(A-120)-60A insert miR-126 | AAAAAAAAAAAAAAAAAAAAAAAAAAAAAAAAAAAAAAAAAAAA AAAAAAAAAAAAAAAAACGCATTATTACTCACGGTACGAAAAAAAAA AAAAAAAAAAAAAAAAAAAAAAAAAAAAAAAAAAAAAAAAAAAA AAAAAAAA |
| 53 | miR-148a binding site | ACAAAGTTCTGTAGTGCACTGA |
| 54 | Poly(A)-0A insert miR-142+miR-126+miR-148a | TCCATAAAGTAGGAAACACTACACGCATTATTACTCACGGTACGAAC AAAGTTCTGTAGTGCACTGAAAAAAAAAAAAAAAAAAAAAAAAAAA AAAAAAAAAAAAAAAAAAAAAAAAAAAAAAAAAAAAAGATATCAAA AAAAAAAAAAAAAAAAAGAAAAAAAAAAAAAAAAAAAAGAAAAAAA AAAAAAAAAA |
| 55 | Poly(A)-0A insert miR-142+miR-148a+miR-126 | TCCATAAAGTAGGAAACACTACAACAAAGTTCTGTAGTGCACTGAC GCATTATTACTCACGGTACGAAAAAAAAAAAAAAAAAAAAAAAAAA AAAAAAAAAAAAAAAAAAAAAAAAAAAAAAAAAAAGATATCAAA AAAAAAAAAAAAAAAAGAAAAAAAAAAAAAAAAAAAGAAAAAAA AAAAAAAAAA |
| 56 | Poly(A)-0A insert miR-126+miR-142+miR-148a | CGCATTATTACTCACGGTACGATCCATAAAGTAGGAAACACTACAAC AAAGTTCTGTAGTGCACTGAAAAAAAAAAAAAAAAAAAAAAAAAAAAA AAAAAAAAAAAAAAAAAAAAAAAAAAAAAAAAAAAAAAAGATATCAAA AAAAAAAAAAAAAAAAAAGAAAAAAAAAAAAAAAAAAAAAGAAAAAAA AAAAAAAAAAA |
| 57 | Poly(A)-0A insert miR-126+miR-148a+miR-142 | *CGCATTATTACTCACGGTACGA*ACAAAGTTCTGTAGTGCACTGATCCA TAAAGTAGGAAACACTACAAAAAAAAAAAAAAAAAAAAAAAAAAAAA AAAAAAAAAAAAAAAAAAAAAAAAAAAAAAAAGATATCAAA AAAAAAAAAAAAAAAAGAAAAAAAAAAAAAAAAAAAGAAAAAAA AAAAAAAAAA |
| 58 | Poly(A)-0A insert miR-148a+miR-142+miR-126 | ACAAAGTTCTGTAGTGCACTGATCCATAAAGTAGGAAACACTACAC GCATTATTACTCACGGTACGAAAAAAAAAAAAAAAAAAAAAAAAA AAAAAAAAAAAAAAAAAAAAAAAAAAAAAAAAAAAAGATATCAAA AAAAAAAAAAAAAAAAAAGAAAAAAAAAAAAAAAAAAAAAGAAAAAAA AAAAAAAAAA |
| 59 | Poly(A)-0A insert | ACAAAGTTCTGTAGTGCACTGACGCATTATTACTCACGGTACGATCCA |
| | miR-148a+miR-126+miR-142 | TAAAGTAGGAAACACTACAAAAAAAAAAAAAAAAAAAAAAAAAA AAAAAAAAAAAAAAAAAAAAAAAAAAAAAAAAAAAGATATCAAA AAAAAAAAAAAAAAAAGAAAAAAAAAAAAAAAAAAAGAAAAAA AAAAAAAAAA |
| 60 | Element d | GATATC |
| 61 | Element d | GTATAC |
| 62 | Element d | GAATCT |
| 63 | Element d | GCATATGACT |
| 64 | Element d | GATATCGTATAC |
| 65 | Poly(A-60)-0A insert miR-142 | TCCATAAAGTAGGAAACACTACAAAAAAAAAAAAAAAAAAAAAAA AAAAAAAAAAAAAAAAAAAAAAAAAAAAAAAAAAAAAA |
| 66 | Poly(A-60)-30A insert miR-142 | AAAAAAAAAAAAAAAAAAAAAAAAAAAAAATCCATAAAGTAGGA AACACTACAAAAAAAAAAAAAAAAAAAAAAAAAAAAAAA |
| 67 | A30LA70 (US10717982B2) | AAAAAAAAAAAAAAAAAAAAAAAAAAAAAGCATATGACTAAAA AAAAAAAAAAAAAAAAAAAAAAAAAAAAAAAAAAAAAAAAAAA AAAAAAAAAAAAAAAAAAAAAA |
| 68 | Poly(A30LA70)-0A insert miR-142 | TCCATAAAGTAGGAAACACTACAAAAAAAAAAAAAAAAAAAAAAA AAAAAAAAGCATATGACTAAAAAAAAAAAAAAAAAAAAAAAAAAA AAAAAAAAAAAAAAAAAAAAAAAAAAAAAAAAAAAAAAAAAAAAA |
| 69 | Poly(A30LA70)-30A insert miR-142 | AAAAAAAAAAAAAAAAAAAAAAAAAAAAAATCCATAAAGTAGGA AACACTACAGCATATGACTAAAAAAAAAAAAAAAAAAAAAAAAA AAAAAAAAAAAAAAAAAAAAAAAAAAAAAAAAAAAAAAAAAAAA |
| 70 | Poly(A30LA70)-60A insert miR-142 | AAAAAAAAAAAAAAAAAAAAAAAAAAAAAAGCATATGACTAAAA AAAAAAAAAAAAAAATCCATAAAGTAGGAAACACTACAAAAAAA AAAAAAAAAAAAAAAAAAAAAAAAAAAAAAAAAAAAAAAAAAAA |
| 71 | Poly(A30LA70)-90A insert miR-142 | AAAAAAAAAAAAAAAAAAAAAAAAAAAAGCATATGACTAAAA AAAAAAAAAAAAAAAAAAAAAAAAAAAAAAAAAAAAAAAAAA AATCCATAAAGTAGGAAACACTACAAAAAAAAAAAAAAAAAAA |
| 72 | 5' UTR | ACTCTTCTGGTCCCCACAGACTCAGAGAGAACCCACC |
| 73 | Luciferase coding region | ATGGAAGACGCCAAAAACATTAAGAAGGGCCCAGCGCCATTCTAC CCACTCGAAGACGGGACCGCCGGCGAGCAGCTGCACAAAGCCATG AAGCGCTACGCCCTGGTGCCCGGCACCATCGCCTTTACCGACGCAC ATATCGAGGTGGACATTACCTACGCCGAGTACTTCGAGATGAGCGT TCGGCTGGCAGAAGCTATGAAGCGCTATGGGCTGAATACAAACCAT CGGATCGTGGTGTGCAGCGAGAATAGCTTGCAGTTCTTCATGCCCG TGTTGGGTGCCCTGTTCATCGGTGTGGCTGTGGCCCCAGCTAACGAC ATCTACAACGAGCGCGAGCTGCTGAACAGCATGGGCATCAGCCAGC CCACCGTCGTATTCGTGAGCAAGAAAGGGCTGCAAAAGATCCTCAA CGTGCAAAAGAAGCTACCGATCATACAAAAGATCATCATCATGGAT AGCAAGACCGACTACCAGGGCTTCCAAAGCATGTACACCTTCGTGA CTTCCCATTTGCCACCCGGCTTCAACGAGTACGACTTCGTGCCCGAG AGCTTCGACCGGGACAAAACCATCGCCCTGATCATGAACAGTAGTG GCAGTACCGGATTGCCCAAGGGCGTAGCCCTACCGCACCGCACCGC TTGTGTCCGATTCAGTCATGCCCGCGACCCCATCTTCGGCAACCAGA TCATCCCCGACACCGCTATCCTCAGCGTGGTGCCATTTCACCACGGC TTCGGCATGTTCACCACGCTGGGCTACTTGATCTGCGGCTTTCGGGT CGTGCTCATGTACCGCTTCGAGGAGGAGCTATTCTTGCGCAGCTTGC AAGACTATAAGATTCAATCTGCCCTGCTGGTGCCCACACTATTTAGC TTCTTCGCTAAGAGCACTCTCATCGACAAGTACGACCTAAGCAACT TGCACGAGATCGCCAGCGGCGGGGCGCCGCTCAGCAAGGAGGTAG |
| | | GTGAGGCCGTGGCCAAACGCTTCCACCTACCAGGCATCCGCCAGGG CTACGGCCTGACAGAAACAACCAGCGCCATTCTGATCACCCCCGAA GGGGACGACAAGCCTGGCGCAGTAGGCAAGGTGGTGCCCTTCTTCG AGGCTAAGGTGGTGGACTTGGACACCGGTAAGACACTGGGTGTGA ACCAGCGCGGCGAGCTGTGCGTCCGTGGCCCCATGATCATGAGCGG CTACGTTAACAACCCCGAGGCTACAAACGCTCTCATCGACAAGGAC GGCTGGCTGCACAGCGGCGACATCGCCTACTGGGACGAGGACGAG CACTTCTTCATCGTGGACCGGCTCAAAAGCCTGATCAAATACAAGG GCTACCAGGTAGCCCCAGCCGAACTGGAGAGCATCCTGCTGCAACA CCCCAACATCTTCGACGCCGGGGTCGCCGGCCTGCCCGACGACGAT GCCGGCGAGCTGCCCGCCGCAGTCGTCGTGCTGGAACACGGTAAAA CCATGACCGAGAAGGAGATCGTGGACTATGTGGCCAGCCAGGTTAC AACCGCCAAGAAGCTGCGCGGTGGTGTTGTGTTCGTGGACGAGGTG CCTAAAGGACTGACCGGCAAGTTGGACGCCCGCAAGATCCGCGAG ATTCTCATTAAGGCCAAGAAGGGCGGAAAGATCGCCGTGTAA |

## Claims

1. An engineered polyadenylic acid (Poly(A)) tail, comprising a miRNA binding site.

2. The engineered Poly(A) tail according to claim 1, comprising or being a structure of Formula (I) below:
nA-miRNA binding location -mA Formula (I),
wherein the miRNA binding location is formed by one or more miRNA binding site linked by one or more nucleotides or directly;
nA represents consecutive n adenylic acids (As) adjacent to the 5' end of the miRNA binding location;
mA represents consecutive m adenylic acids (As) adjacent to the 3' end of the miRNA binding location;
m and n are natural numbers, and m+n≤150, m+n≤120, m+n≤100, m+n≤80, m+n≤60, m+n≤30, m+n≤19, or m+n≤14, and preferably, where m+n=60; and
the 3' and 5' ends of the miRNA binding location comprise no other As than As comprised in the miRNA binding site.

3. The engineered Poly(A) tail according to claim 2, wherein n=0 or n≥1.

4. The engineered Poly(A) tail according to claim 2, wherein n≤60, n≤30, n≤19, n≤14, or n≤10.

5. The engineered Poly(A) tail according to claim 2, wherein 14≤n≤30, and preferably 19≤n≤30 or 14≤n≤19.

6. The engineered Poly(A) tail according to any one of claims 1 to 5, having a length of 80 to 240 nt, for example, 100 to 200 nt, 101 to 150 nt, 120 to 150 nt, 130 to 140 nt, 123 to 135 nt, or 125 to 139 nt.

7. The engineered Poly(A) tail according to any one of claims 2 to 6,
further comprising, at the 3' side of the structure of Formula (I), a tail fragment directly linked to the 3' end of the structure of Formula (I), wherein the 5'-terminal nucleotide of the tail fragment is not A; and/or
further comprising, at the 5' side of the structure of Formula (I), a head fragment directly linked to the 5' end of the structure of Formula (I), where the 3'-terminal nucleotide of the head fragment is not A.

8. The engineered Poly(A) tail according to claim 7, wherein the tail fragment or the head fragment comprises one or more elements c and/or one or more elements d and one or more As, wherein
element c is a non-A nucleotide,
element d comprises any two or more consecutive nucleotides, the 5'- and 3'-terminal nucleotides of element d are not A, and element d does not comprise more than three consecutive As;
element d has a length in the range of 2 nt≤d≤20 nt; and
element c and element d are not contiguous.

9. The engineered Poly(A) tail according to claim 8, wherein the number of element c comprised is 2 to 10, 3 to 8, 4 to 6, or 2 to 5, and preferably 2; and/or element c is G.

10. The engineered Poly(A) tail according to any one of claims 8 to 9, wherein element d is any one or more selected from the sequences as shown in SEQ ID NOs: 60 to 64, and preferably, element d is as shown in SEQ ID NO: 60.

11. The engineered Poly(A) tail according to any one of claims 8 to 10, wherein the number of element d comprised is 0-5, preferably 1 to 3, and further preferably 1.

12. The engineered Poly(A) tail according to any one of claims 1 to 11, comprising no head fragment, wherein the tail fragment has a structure of: element d-19A-element c-19A-element c-17A or element c-19A-element c-17A.

13. The engineered Poly(A) tail according to any one of claims 1 to 12, having a structure of nA-miRNA binding location -mA-element d-19A-element c-19A-element c-17A.

14. The engineered Poly(A) tail according to any one of claims 1 to 13, having a structure of nA-miRNA binding location -mA-SEQ ID NO: 60-19A-G-19A-G-17A.

15. The engineered Poly(A) tail according to any one of claims 1 to 14, wherein the miRNA is one or more selected from:
miR-142, miR-122, miR-126, miR-148a, miR-133, miR-206, miR-208, miR-17-92, miR-16, miR-21, miR-223, miR-24, miR-27, let-7, miR-30c, miR-1d, miR-149, miR-192, miR-194, miR-101 and miR-204;
preferably, the miRNA is one or more selected from miR-142, miR-122, miR-126, and miR-148a; and
preferably, the miRNA is one or more selected from: a combination of miR-142 and miR-122, a combination of miR-126 and miR-148a, and a combination of miR-142, miR-148a, and miR-126.

16. The engineered Poly(A) tail according to any one of claims 1 to 15, wherein the miRNA comprises miRNA-142, miR-148a and miR-126; and in the miRNA binding location of the Poly(A), the miRNA binding sites are arranged, from 5' to 3', in a sequence of:
miR-126 binding site, miR-148a binding site and miR-142 binding site;
miR-148a binding site, miR-142 binding site and miR-126 binding site;
miR-142 binding site, miR-148a binding site and miR-126 binding site;
miR-126 binding site, miR-142 binding site and miR-148a binding site;
miR-148a binding site, miR-126 binding site and miR-142 binding site; or
miR-142 binding site, miR-126 binding site and miR-148a binding site,
wherein the miR-126 binding site, the miR-148a binding site, and the miR-142 binding site are adjacent to each other.

17. The engineered Poly(A) tail according to any one of claims 1 to 16, wherein n=0, m=60, and mA and the tail fragment form a polynucleotide sequence as shown in SEQ ID NO: 1.

18. The engineered Poly(A) tail according to any one of claims 1 to 16, wherein the Poly(A) tail comprises any polynucleotide sequence selected from:
SEQ ID NO: 6-16, SEQ ID NO: 19-31, SEQ ID NO: 33-52, SEQ ID NO: 54-59 and SEQ ID NO: 65-71.

19. An engineered RNA molecule, which is an mRNA or a non-coding RNA, and comprising the Poly(A) tail according to any one of claims 1 to 18 and a target gene sequence located at the 5' side of the Poly(A) tail, wherein the RNA sequence comprises, from the 5' to 3' end, at least a 5'UTR sequence, the target gene sequence, a 3'UTR sequence, and the Poly(A) tail according to any one of claims 1 to 18 in sequence, wherein nA in the structure of Formula (I) in the Poly(A) tail is directly linked to 3'UTR, and preferably, the RNA molecule has a 5' cap and/or all or some of uridylic acid is modified into N1- methyl pseudouridine.

20. An engineered DNA molecule, encoding the engineered Poly(A) tail according to any one of claims 1 to 18, or the engineered RNA sequence according to claim 19, wherein preferably, the DNA molecule is a plasmid or viral vector.

21. An engineered cell, comprising the engineered Poly(A) tail according to any one of claims 1 to 18, the engineered RNA molecule according to claim 19, or the engineered DNA molecule according to claim 20, wherein the cell is an eukaryotic or a prokaryotic cell; and preferably the prokaryotic cell is *E. coli.*

22. Use of the engineered Poly(A) tail according to any one of claims 1 to 18 in regulating the expression of a target gene in a particular organ, tissue and/or cell, wherein the particular organ, tissue and/or cell highly expresses the miRNA, and preferably the regulation is to enable low expression of the target gene in the particular organ, tissue and/or cell; and more preferably, the particular organ, tissue and/or cell is a liver or spleen organ, tissue and/or cell.

23. Use of the engineered Poly(A) tail according to any one of claims 1 to 18 in enabling the specific expression of a target gene in a particular organ, tissue and/or cell, wherein preferably, the organ or tissue is preferably a liver or spleen organ or tissue; and further preferably the cell is hepatic parenchymal cells.

24. Use of the Poly(A) tail according to any one of claims 1 to 18 in enabling the more conservative replication of a DNA coding sequence encoding the Poly(A) tail in a host cell, wherein preferably, the host cell is a prokaryotic cell, and further preferably *E. coli.*

25. A lipid nanoparticle, comprising the engineered Poly(A) tail according to any one of claims 1 to 18, the engineered RNA molecule according to claim 19, or the engineered DNA molecule according to claim 20.

26. The lipid nanoparticle according to claim 25, comprising an ionizable lipid, a phospholipid, a structural lipid and a PEGylated lipid, wherein preferably, the molar ratio of the ionizable lipid, the phospholipid, the structural lipid and the PEGylated lipid is: (20-60): (5-25): (25-55): (0.5-15), and further preferably (40-55): (5-15): (30-50): (1-3);
the phospholipid is preferably one or more selected from the following compounds:
dilauroyl-sn-glycero-phosphocholine (DLPC),
dimyristoyl-sn-glycero-phosphocholine (DMPC),
dioleoyl-sn-glycero-phosphocholine (DOPC),
dipalmitoylphosphatidylcholine (DPPC),
distearoyl-sn-glycero-3-phosphocholine (DSPC),
diundecanoyl-sn-glycero-phosphocholine (DUPC),
palmitoyl-oleoyl-glycero-phosphocholine (POPC),
1,2-di-O-octadecyl-sn-glycero-3-phosphocholine (18:0 Diether PC),
1-oleoyl-2-cholesterylhemisuccinoyl-sn-glycero-3-phosphocholine (OChemsPC),
1-hexadecyl-sn-glycero-3-phosphocholine (C16 Lyso PC),
1,2-divinyl-sn-glycero-3-phosphocholine,
1,2-diarylacyl-sn-glycero-3-phosphocholine,
1,2-dioleoyl-SN-glycero-3-phosphoethanolamine (DOPE),
1,2-distearoyl-sn-glycero-3-phosphoethanolamine,
1,2-divinylol-sn-glycero-3-phosphoethanolamine,
1,2-divinyl-sn-glycero-3-phosphoethanolamine,
1,2-diaryl-sn-glycero-3-phosphoethanolamine,
1,2-dithiohexaenoic acid-sn-glycero-3-phosphoethanolamine,
1,2-dioleoyl-sn-glycero-3-phosphatidyl-(1-glycerol) sodium salt (DOPG) or sphingomyelin,
for example, the phospholipid is DOPE or DSPC;
the structural lipid is preferably one or two or more selected from cholesterol, coprosterol, sitosterol, ergosterol and stigmasterol; for example, the structural lipid is cholesterol; and/or
the PEGylated lipid is preferably one or more selected from PEG-modified phosphatidylethanolamine, PEG-modified phosphatidic acid, PEG-modified ceramide, PEG-modified dialkyl amine, PEG-modified diacylglycerol or PEG-modified dialkyl glycerol, for example, the PEGylated lipid is DMG-PEG2000.
